# EUROPÄISCHE PATENTANMELDUNG

(11) **EP 3 910 646 A1**
(43) Veröffentlichungstag der Anmeldung: **17.11.2021**
(21) Anmeldenummer: 20173956.2
(22) Anmeldetag: 11.05.2020
(51) Int. Cl.: G16H 40/20, G06Q 10/06, G06Q 50/00, G06F 21/31, G07C 9/32

(54) **MEDIZINISCHES BEHANDLUNGSSYSTEM ZUR IDENTIFIZIERUNG UND AUTORISIERUNG MEDIZINISCHER KRAEFTE**

(71) Anmelder: Fresenius Medical Care Deutschland GmbH, 61352 Bad Homburg (DE)
(72) Erfinder: Müller, Carsten, 97502 Euerbach (DE); Thorwarth, Michael, 60486 Frankfurt am Main (DE); Speth, Timo, 97525 Schwebheim (DE); Trauner, Karin Helga, 97422 Schweinfurt (DE); Müller, Ralf, 61348 Bad Homburg (DE); Miao, Qingsong, 60326 Frankfurt am Main (DE)
(74) Vertreter: Bobbert & Partner Patentanwälte PartmbB

(57) **Zusammenfassung**

Die vorliegende Erfindung betrifft ein medizinisches Behandlungssystem (5000), mit einer Lesevorrichtung (A) programmiert zum - vorzugsweise kontaktlosen - Erfassen von Informationen, welche einer Identifizierung einer medizinischen Kraft (U) dienen, und programmiert zum Bereitstellen der erfassten Informationen an eine Berechtigungsvorrichtung. Ferner weist das medizinische Behandlungssystem (5000) eine Vorrichtung aus einer Gruppe von Elementen auf, welche aus einer oder mehreren Behandlungsvorrichtungen (1000) zum Behandeln eines Patienten, einer oder mehreren Diagnosevorrichtungen (2000) zum Untersuchen des Patienten und einer oder mehreren weiteren Klinikvorrichtungen (3000), z. B. zum logistischen Ablauf des Klinik- oder Praxisbetriebs, besteht. Außerdem weist das medizinischen Behandlungssystem (5000) eine Berechtigungsvorrichtung (B) zum Erlauben oder Verbieten von einer oder mehreren Tätigkeiten mit oder an der Vorrichtung der Gruppe und/oder einer oder mehreren Bewegungen der medizinischen Kraft (U) im Umfeld der Vorrichtung aus der Gruppe in Abhängigkeit der mittels der Lesevorrichtung (A) bereitgestellten Informationen.

## Beschreibung

Die vorliegende Erfindung betrifft ein medizinisches Behandlungssystem gemäß Anspruch 1.

Das moderne Gesundheitssystem ermöglicht eine nicht gekannte medizinische Versorgung heutiger Patienten, nicht zuletzt durch den Einsatz komplexer Diagnose- und Behandlungsvorrichtungen. Ihre ordnungsgemäße Bedienung erfordert besonderes medizinisches aber auch technisches Wissen. Patientensicherheit kann im Zusammenhang mit solchen komplexen Diagnose- und Behandlungsvorrichtungen zumeist nur gewährleistet werden, wenn sichergestellt ist, dass unberechtigte Personen von der beabsichtigten oder unbeabsichtigten Bedienung solcher Vorrichtungen ausgeschlossen sind, oder wenn sichergestellt ist, dass dieser Personenkreis gar nicht erst Zugang zu solchen oder anderen medizinischen Vorrichtungen hat.

Als Beispiel einer komplexen Behandlungsvorrichtung aus der Praxis sei eine Vorrichtung zur extrakorporalen Blutbehandlung mittels Dialyse genannt. Bei einer solchen Blutbehandlung wird dem Patienten Blut entnommen, das entlang eines Blutkreislaufs extrakorporal und durch einen Blutfilter der Dialysevorrichtung geführt wird. Während der so durchgeführten Blutbehandlungssitzung kann über eine Vielzahl von an der Dialysevorrichtung einstellbaren Behandlungsparametern Einfluss auf die Behandlung genommen werden, unter anderem durch die Änderung von Flussraten und/oder von Medikamentendosierungen. Es bedarf keiner Erläuterung, dass derartige Einstellungen vorteilhafterweise nur von einem ausgewählten, ausreichend qualifizierten Personenkreis vorgenommen werden sollten.

Aber auch bereits der Zutritt Unberechtigter zu Patientenzimmern, Behandlungsräumen, Isolierstationen, medizinischen Vorrichtungen und dergleichen kann, sowohl aus Gründen des Schutzes des Unberechtigten (Stichwort: Isolierstation) als auch zum Schutz des Patienten (Stichwort: Transplantationsstation) aber auch aus Gründen der Privatsphäre des Patienten und/oder des Datenschutzes, streng begrenzt sein.

Es ist daher erstrebenswert, medizinische Behandlungssysteme, also v. a. Vorrichtungen zum Behandeln oder Untersuchen von Patienten, und deren Aufstellorte, wie z. B. Klinik- oder Praxisräume, vor dem Zugriff oder Zugang Unberechtigter zu schützen.

Eine Aufgabe der vorliegenden Erfindung kann darin gesehen werden, ein solches medizinisches Behandlungssystem vorzuschlagen.

Die erfindungsgemäße Aufgabe wird durch ein medizinisches Behandlungssystem (oder "medizinisches Set") mit den Merkmalen des Anspruchs 1 gelöst.

Die vorliegende Erfindung betrifft ein medizinisches Behandlungssystem, welches eine Lesevorrichtung, eine medizinische Vorrichtung und eine Berechtigungsvorrichtung umfasst.

Die Lesevorrichtung ist vorzugsweise als Einlese- und/oder als Auslesevorrichtung ausgestaltet. Sie ist programmiert, um - vorzugsweise kontaktlos - Daten oder Informationen (im Folgenden auch kurz: Informationen) zu erfassen. Dabei kann das Erfassen ein Einlesen oder Auslesen der Informationen sein.

Die Informationen, die von der Lesevorrichtung erfasst werden, dienen einer Identifizierung von Klinikpersonal, hierin auch als eine medizinische Kraft bezeichnet, z. B. der Identifizierung einer Ärztin, eines Pflegers, eines Technikers oder einer anderen Person aus dem Umfeld der Klinik oder Praxis, wobei hierin als medizinische Kraft insbesondere auch Patienten oder Besucher zählen können.

Ferner ist die Lesevorrichtung programmiert, um die erfassten Informationen bereitzustellen, beispielsweise, um die Informationen an die nachfolgend weiter beschriebene Berechtigungsvorrichtung zu übermitteln oder die Informationen von der Berechtigungsvorrichtung auslesen zu lassen oder dieser auf andere Weise zur Verfügung zu stellen oder dieser zu übermitteln.

Identifizieren bedeutet hierin insbesondere, eine Person ausreichend genau, d. h. möglichst eindeutig, wiederzuerkennen, d. h. ihre Identität und/oder Echtheit festzustellen, wie dies in der Praxis Ziel z. B. auch von Passkontrollen ist. "Identifizieren" wird hierin synonym zu "authentifizieren" und "erkennen" gebraucht. Diese Begriffe sind somit austauschbar. "Autorisieren" ist synonym zu der einer Identifikation nachgestellten Ermittlung einer Berechtigung. Hierin geht die Identifizierung/Authentifizierung stets vorrangig einer nachfolgenden Autorisierung voraus. Bedeutungsunterschiede sind eindeutig dem jeweiligen Kontext zu entnehmen.

Das Identifizieren kann mittels der Lesevorrichtung erfolgen.

Die medizinische Vorrichtung ist ein Element aus einer Gruppe von Elementen, welche aus einer oder mehreren Behandlungsvorrichtungen zum Behandeln des Patienten, einer oder mehreren Diagnosevorrichtungen zum Untersuchen des Patienten und einer oder mehreren weiteren Klinikvorrichtungen, z. B. zum logistischen Ablauf des Klinik- oder Praxisbetriebs, besteht.

Die vorstehend bereits angesprochene Berechtigungsvorrichtung ist programmiert oder konfiguriert zum Erlauben oder Verbieten von einer oder mehreren Tätigkeiten mit oder an der medizinischen Vorrichtung aus der oben genannten Gruppe und/oder einer oder mehreren Bewegungen der medizinischen Kraft im Umfeld der medizinischen Vorrichtung aus der oben genannten Gruppe, insbesondere mittels Aktivieren oder Blockieren von Klinikvorrichtungen, beispielsweise Türen. Das Erlauben oder Verbieten erfolgt in Abhängigkeit vom Ergebnis einer Auswertung der Informationen, die mittels der Lesevorrichtung bereitgestellt wurden. Die Auswertung kann bereits durch die Lesevorrichtung, alternativ z. B. durch die Berechtigungsvorrichtung erfolgen.

Das Erlauben kann ein Ermöglichen, ein Autorisieren oder Gestatten einer Tätigkeit oder Bewegung sein, ebenso ein Entsperren und/oder ein Aufschließen z. B. einer Vorrichtung wie einer Tür, eines Schlosses, eines Computerzugangs, usw. Autorisieren bedeutet, wie hierin verwendet, jemanden zu bevollmächtigen, hier eine Tätigkeit oder Bewegung auszuführen, ihn zu ermächtigen oder ihm eine Genehmigung für etwas, hier die Tätigkeit oder Bewegung, zu erteilen.

Das Verbieten kann ein Nicht-Ermöglichen, ein Versagen, ein Nicht-Autorisieren oder ein Nicht-Gestatten sein, ebenso ein Sperren, ein Ausschließen und/oder ein Verschließen.

Bewegungen können ein Bewegen oder Fortbewegen der medizinischen Kraft in einer Richtung, ein Betreten eines Bereichs durch die medizinische Kraft, ihr Eintreten in einen Raum und/oder ihr anderweitiges Fortbewegen, insbesondere in Zusammenhang mit der medizinischen Vorrichtung oder deren Position sein. Das Erlauben oder Verbieten von Bewegungen kann als das Gewähren oder Versagen von Bewegungsfreiheit verstanden werden, jedenfalls zu einem vorbestimmten Ort oder Raum und/oder in einen vorbestimmten Ort oder Raum hinein.

Tätigkeiten können das Bedienen oder Benutzen der medizinischen Vorrichtung umfassen.

Die Berechtigungsvorrichtung kann somit zu Tätigkeiten an der medizinischen Vorrichtung berechtigen, z. B. zum Starten, Einstellen oder Abändern eines vorbestimmten, mittels der medizinischen Vorrichtung durchzuführenden oder durchgeführten Arbeitsablaufs (*workflow*).

Die Berechtigungsvorrichtung kann sich erfindungsgemäß zum Erlauben oder Verbieten von Tätigkeiten oder Bewegungen geeigneter Aktoren wie Passwort-geschützte Bildschirmschoner, Softwareprogramme oder Touchscreen- Oberflächen, Schlösser, Türöffner, usw. bedienen, von denen hier nur einige exemplarisch genannt sind.

Wenn hierin von "programmiert" oder "konfiguriert" die Rede ist, so ist auch offenbart, diese Begriffe gegeneinander auszutauschen.

Wenn hierin von "programmiert" oder "konfiguriert" die Rede ist, so ist auch offenbart, dass die betreffende Vorrichtung in einigen Ausführungsformen zum automatischen Ausführen von Schritten, zu welchen sie programmiert oder konfiguriert ist, eingerichtet ist.

Wenn hierin ein Schritt oder eine Handlung offenbart ist, so ist auch offenbart, dass jeweils wenigstens eine Vorrichtung des medizinischen Behandlungssystems in manchen Ausführungsformen zum Ausführen des Schritts oder der Handlung programmiert oder konfiguriert ist.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen vorstehenden oder folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt diese stets eine erfindungsgemäße, beispielhafte Ausführungsform dar.

In einigen Ausführungsformen ist oder umfasst die Lesevorrichtung eine Sensoranordnung, insbesondere einen Kartenleser, einen Radarsender und/oder -empfänger, eine Kamera, usw. oder beliebige Kombinationen dieser und aller weiteren hierin genannten Sensoren, Scanner, Empfänger, Leser und andere Einrichtungen oder Vorrichtungen.

In manchen Ausführungsformen ist oder umfasst die Lesevorrichtung ein(en) Fingerabdruckscanner zum Scannen von Fingerabdrücken, eine Einrichtung zum Erkennen eines Bewegungsmusters, von Gesten, von biometrischen Daten oder Signaturen oder der Irisstruktur. Zu biometrischen Daten oder Signaturen zählen beispielsweise unter anderem Fingerabdrücke, Gesichtszüge, Irisstrukturen, Bewegungsmuster und/oder Sprachsignaturen.

Die Lesevorrichtung ist hierzu vorzugsweise ausgestaltet als Kamera, als Sender und/oder Empfänger von Radarwellen, als Kartenleser für Magnet- oder Chipkarten, als Mikrofon zum Erfassen und/oder Analysieren der Stimme der medizinischen Kraft (vorzugsweise mit entsprechender Spracherkennung), als Eingabevorrichtung konfiguriert zum Eingeben von persönlichem Wissen (mittels Displays oder Monitors, mittels haptischer Eingabevorrichtungen, beispielsweise wenigstens eines Touchpads oder wenigstens einer Tastatur), oder weist dergleichen auf.

Daten oder Informationen, die auf der Identifizierungsvorrichtung gespeichert sind, welche die medizinische Kraft optional mit sich führt, werden vorzugsweise mittels Kamera oder anderen hierzu geeigneten und programmierten Erkennungsvorrichtungen oder Empfangsvorrichtungen der Lesevorrichtung erfasst und/oder mit Hilfe von WiFi, Bluetooth oder RFID übertragen, insbesondere wie sie hierin offenbart sind.

Mittels vorstehender Ausgestaltungen der Lesevorrichtung im Gebrauch des medizinischen Behandlungssystems erfasste Informationen können hierin jene Daten und Informationen (kurz: Informationen) verstanden werden, welche der Identifizierung einer medizinischen Kraft dienen.

Solche Daten und Informationen können auf wenigstens einem Server, einer Datenbank und/oder einem Datenspeicher gespeichert werden und/oder sein und/oder mit Daten auf wenigstens einem Server einer Datenbank und/oder einem Datenspeicher verglichen werden. Eine Datenbank kann sich auf einem dezidierten Datenbankserver, auf einem Personal Computer oder einer anderen Vorrichtung befinden. Die Daten können unverschlüsselt oder verschlüsselt gespeichert werden und/oder sein.

In manchen Ausführungsformen ist die Lesevorrichtung kabelgebunden und/oder drahtlos mit wenigstens einem Server, einer Datenbank und/oder einem Datenspeicher verbunden.

In einigen Ausführungsformen ist die Berechtigungsvorrichtung kabelgebunden und/oder drahtlos mit wenigstens einem Server, einer Datenbank und/oder einem Datenspeicher verbunden.

In manchen Ausführungsformen findet die Verbindung der Lesevorrichtung und/oder der Berechtigungsvorrichtung und/oder einer anderen hierin genannten Vorrichtung und/oder Einrichtung mit dem wenigstens einem Server, einer Datenbank und/oder einem Datenspeicher über wenigstens ein Netzwerk kabelgebunden oder drahtlos statt.

Radar, soweit hier genannt, ist die Abkürzung für *Radio Detection and Ranging* (etwa: funkgestützte Ortung und Abstandsmessung). Unter diesem Begriff werden verschiedene Erkennungs- und Ortungsverfahren für Gegenstände (auch Personen) auf der Basis elektromagnetischer Wellen im Radiofrequenzbereich (Funkwellen) zusammengefasst. Soweit für den Fachmann nicht erkennbar technisch unmöglich, ist bei dem hier genannten Radar auch Lidar (*Light Detection and Ranging*) auf Basis von Laserstrahlen zu verstehen.

Der Radarsender des Radargeräts sendet eine gebündelte elektromagnetische Welle als sogenanntes Primärsignal aus. Diese werden von Objekten reflektiert und die Echos vom Radarempfänger des Radargeräts als sogenanntes Sekundärsignal empfangen. Die empfangenen Echos können nach vorbestimmten Kriterien ausgewertet werden. Unter anderem können dabei folgende Informationen gewonnen werden:
- Winkel oder Richtung zum (reflektierenden) Objekt (oder: Subjekt);
- Entfernung zum Objekt (mittels der Zeitverschiebung zwischen Senden und Empfangen);
- Relativbewegung zwischen Sender und Objekt (mittels Doppler-Effekt aus der Frequenzverschiebung);
- Absolute Geschwindigkeit des Objekts (mittels Aneinanderreihens mehrerer einzelner Messungen);
- Konturen oder Bilder des Objekts.

In manchen Ausführungsformen ist die Lesevorrichtung programmiert, um ein Gangmuster, Gangschema, Bewegungsprofil, die Gesten oder dergleichen der medizinischen Kraft als Information(en) zu erfassen.

In einigen Ausführungsformen weist das medizinische Behandlungssystem weiter eine Identifizierungsvorrichtung auf. Die Identifizierungsvorrichtung ist programmiert und/oder konfiguriert, um am Körper der medizinischen Kraft getragen oder von dieser mitgeführt zu werden.

Weist das medizinische Behandlungssystem also auch eine Identifizierungsvorrichtung auf, so kann die Lesevorrichtung in solchen Ausführungsformen programmiert sein, Informationen, welche auf der Identifizierungsvorrichtung gespeichert sind, von dieser zu erfassen und/oder zu übermitteln. Alternativ können relevante Informationen bereits dadurch erfasst werden, dass festgestellt wird, dass die medizinische Kraft eine ganz konkrete, vorbestimmte Identifizierungsvorrichtung oder eine Identifizierungsvorrichtung einer vorbestimmten Ausprägung oder Typ mit sich trägt, gleich ob diese Identifizierungsvorrichtung Informationen bezüglich der Identität der konkreten, sie mit sich tragenden medizinischen Kraft bereithält oder nicht.

In einigen Ausführungsformen ist die Lesevorrichtung konfiguriert, eine von der medizinischen Kraft mit sich geführte Identifizierungsvorrichtung beispielsweise mittels Radartechnik auszulesen oder auch nur deren Vorhandensein festzustellen. In manchen Ausführungsformen bedarf es zum Erfassen der Information bezüglich der Identität der medizinischen Kraft mittels Radartechnik keiner mitgeführten Identifizierungsvorrichtung.

In manchen Ausführungsformen ist die Lesevorrichtung programmiert, um die erfasste Informationen, insbesondere solche Informationen, welche durch Auslesen der Identifizierungsvorrichtung erfasst und übermittelt wurden, mit in der Lesevorrichtung oder in einer anderen Speichervorrichtung des medizinischen Behandlungssystems gespeicherten Informationen zu vergleichen. Werden alternativ oder ergänzend biometrische Daten oder Signaturen oder andere Informationen erfasst, welche nicht auf einer Identifizierungsvorrichtung vorliegen, so kann vorgesehen sein, diese mit gespeicherten biometrischen Daten oder Signaturen zu vergleichen.

In einigen Ausführungsformen ist die Lesevorrichtung programmiert, um die Informationen durch Abfragen persönlichen Wissens der medizinischen Kraft zu erfassen und mit in der Lesevorrichtung oder an anderer Stelle des medizinischen Behandlungssystems gespeicherten Informationen zu vergleichen. Persönliches Wissen umfasst beispielsweise eine Benutzername/Passwort-Kombination, andere Kombinationen von - z. B logischen - Zeichenfolgen und/oder Antworten auf persönliche Fragen, beispielweise wie der Mädchenname der Mutter oder der Name des Haustiers lautet.

In manchen Ausführungsformen ist die Lesevorrichtung programmiert, um basierend auf dem Ergebnis des Vergleichens die Identität der medizinischen Kraft festzustellen und/oder sie zu bestimmten Tätigkeiten und/oder Bewegungen zu autorisieren.

Die Informationen, die durch Abfragen persönlichen Wissens oder durch Auslesen der Identifizierungsvorrichtung erfasst wurden, können in einigen Ausführungsformen der vorliegenden Erfindung mit Informationen, welche auf einem Server und/oder in einer Datenbank und/oder einem Datenspeicher (im Folgenden auch kurz Server) vorliegen, verglichen werden. In diesen Ausführungsformen wird die Identität der medizinischen Kraft und/oder die Autorisierung zu bestimmten Tätigkeiten und/oder Bewegungen durch einen positiven Vergleich der Abfrage und/oder des Auslesens mit Informationen, die auf dem Server vorliegen, bestätigt.

Ein ,positiver Vergleich' im Sinne der vorliegenden Erfindung kann die Übereinstimmung der durch die Lesevorrichtung erfassten und/oder abgefragten Informationen mit den beispielsweise auf einem oder mehreren Server(n), einer oder mehreren Datenbank(en) oder einem oder mehreren Datenspeicher(n) gespeicherten Informationen sein.

Wenn hierin die Rede ist von Informationen, die "im Netzwerk" vorliegen und/oder gespeichert und/oder hinterlegt werden, so werden diese Daten vorzugsweise auf einer oder mehreren hierfür geeigneten Vorrichtung(en), beispielsweise einem oder mehreren Server(n), einer oder mehreren Datenbank(en) oder einem oder mehreren Datenspeicher(n), gespeichert, hinterlegt und/oder liegen hierauf vor.

Die Speicherung und/oder das Hinterlegen von Informationen auf beispielsweise einem oder mehreren Server(n), einer oder mehreren Datenbank(en) oder einem oder mehreren Datenspeicher(n) kann derart erfolgen, dass unterschiedliche Informationen, beispielsweise zu Gruppen von medizinischen Kräften oder Bewegungsprofilen oder anderen in der Erfindung genannten Informationen, auf getrennten Servern, Datenbanken und/oder Datenspeichern gespeichert und/oder hinterlegt sind oder werden. Unterschiedliche Informationen können aber auch auf den gleichen beispielsweise einem oder mehreren Server(n), einer oder mehreren Datenbank(en) oder einem oder mehreren Datenspeicher(n) oder jeder möglichen Kombination gespeichert und/oder hinterlegt sein oder werden.

In einigen Ausführungsformen ist die Lesevorrichtung programmiert, um die Identität und/oder eine entsprechende Berechtigung der dieser Identität zugeordneten medizinischen Kraft an die Berechtigungsvorrichtung zu übermitteln. In anderen Ausführungsformen ist die Lesevorrichtung vorgesehen und/oder geeignet, um von der Berechtigungsvorrichtung zu diesem Zweck ausgelesen zu werden.

Berechtigungen, beispielsweise Zugangsberechtigungen oder die Berechtigungen, bestimmte Tätigkeiten auszuführen, die einer medizinischen Kraft erteilt wurden, können jederzeit widerrufen oder beendet werden. Dies kann durch ein Überschreiben oder Ändern von zuvor erteilten Berechtigungen erfolgen, was hierin auch als ein Widerruf zu verstehen ist. Berechtigungen können wie deren Widerruf zeitbasiert erfolgen und/oder abhängig von einem Ort, an dem sich die medizinische Kraft befindet oder welche sie aufsuchen könnte. Zeitbasierte Berechtigungen können beispielsweise für ein bestimmtes Zeitfenster, beispielsweise nach erfolgter Identifizierung, erteilt werden. Denkbar ist hier die Länge einer Arbeitsschicht (meist etwa 8 bis 9 aufeinanderfolgende Stunden) nach der ersten Identifizierung am konkreten Tag, einige Minuten nach der Identifizierung an einer Behandlungsvorrichtung, eine Stunde nach der letzten Identifizierung oder Ähnliches. Für den Widerruf abhängig vom Ort können bestimmte Zonen bestimmt werden, nach deren Betreten, eine oder mehrere Berechtigung(en) widerrufen wird/werden, etwa nach Betreten der Infektionsstation, nach deren Besuch der medizinischen Kraft der bislang für sie vielleicht unbeschränkte Zugang zur Transplantationsstation nicht mehr möglich sein soll, ihr daher die hierfür erforderliche Berechtigung entzogen wird. Diese Zonen können vorzugsweise frei z. B. mittels Softewarelösungen im medizinischen Behandlungssystem oder einer Steuervorrichtung hiervon bestimmt werden. Insbesondere werden diese Zonen beispielsweise durch die Reichweite der Sensoranordnungen (Kamera, Radar) bestimmt, innerhalb welcher die medizinische Kraft selbst oder ihre Identifizierungsvorrichtung erfasst wird.

Ein Widerruf kann ebenfalls vorgesehen sein, wenn die medizinische Kraft in Kontakt mit einer Person gekommen ist, mit der sie zum Beibehalten ihrer Berechtigung(en) nicht hätte kommen dürfen. Dies kann besonders in Zeiten erhöhter Ansteckungsgefahr (Grippewelle, Corona-Welle, Masernwelle, Epidemie, Pandemie etc.) wichtig sein.

So kann es beispielsweise vorgesehen sein, dass ein Patient, der sich vermutlich oder nachweislich mit einer bestimmten Art von Virus oder Bakterium infiziert hat oder aufgrund anderer Umstände als infektiös gilt, diese Information, z. B. mit dem Vermerk "infektiös", auf einer ihm zugeordneten Identifizierungsvorrichtung hinterlegt mit sich trägt. Dies könnte beispielsweise auf einem am Pyjama getragenen Marker als Beispiel einer Identifizierungsvorrichtung des erfindungsgemäßen medizinischen Behandlungssystems erfolgen. Ein solcher Marker kann beliebige Merkmale im Zusammenhang mit der Identifizierungsvorrichtung, wie hierin beschrieben, aufweisen, insbesondere solche, welche den hier beschriebenen Einsatz des Markers (alternativ: Markierungssystems) möglich machen und/oder hierzu beitragen. Mittels der vorliegenden Erfindung kann die Sicherheit im klinischen Umfeld beispielsweise dadurch erhöht werden, dass beispielsweise zum Schutz anderer Patienten und/oder des Pflegepersonals und/oder zur Verhinderung weiterer Verbreitung des Keims im Allgemeinen, mittels der vorliegenden Erfindung festgestellt werden kann, wer sich mit diesem Patienten in einem Raum bzw. in seiner Nähe aufhält, aufgehalten hat oder vor einem Aufenthalt in dessen Nähe gewarnt werden kann.

Betrifft dies medizinische Kräfte, z. B. Ärzte/Ärztinnen oder Pflegepersonal, die noch auf anderen sensiblen Stationen zugegen sind, beispielsweise Neugeborenen- oder Kinderstationen, Intensivstationen, Transplantationsstationen oder dergleichen, so kann von der vorliegenden Erfindung beispielsweise bestimmt sein, dass ein Widerruf von Berechtigungen erfolgt, wenn die Lesevorrichtung oder ein mit ihr verbundener Sensor feststellt, dass sich sowohl die betreffende medizinische Kraft als auch der als "infektiös" markierte Patient - oder eine andere medizinische Kraft, die z. B. zuvor auf der Infektionsstation tätig war - in demselben Raum, vor derselben Kamera, mit weniger Abstand zueinander als dem geforderten Mindestabstand, usw., insbesondere zeitgleich, aufhalten oder aufgehalten haben. Der medizinischen Kraft kann dann anschließend mittels Hinweises und/oder mittels Entzugs von Berechtigungen verwehrt sein, bestimmte Bereiche der Klinik/Praxis zu betreten, zu denen sie vorher noch Zugang hatte.

Wann diese Berechtigung wieder erteilt wird, ob eine zwischenzeitlich durchgeführte (ein- oder mehrfache) Desinfektion, ein Kleiderwechsel oder dergleichen ausreicht oder, ob (im schlimmsten Fall) eine Quarantäne und/oder ein Test auf den Keim nötig ist, bevor die Arbeit wieder aufgenommen oder die entzogene Berechtigung erneut erteilt werden kann, wird von Fall zu Fall entschieden werden müssen, oder z. B. nach vorbestimmten, hinterlegten Regeln. Auch das Hinterlegen solcher "Quarantänezeiten" kann im erfindungsgemäßen medizinischen Behandlungssystem vorgesehen sein. Der entsprechenden medizinischen Kraft kann beispielsweise dann der Zutritt zur Klinik/Praxis ganz verwehrt sein. Vorteilhafterweise wird durch solche Maßnahmen eine Ausbreitung des Keims gehemmt (oder idealerweise verhindert).

Das erfindungsgemäße medizinische Behandlungssystem kann somit mehr als nur eine Identifizierungsvorrichtung aufweisen. Es kann neben Identifizierungsvorrichtungen, welche zum Tragen z. B. durch medizinisches Personal vorgesehen ist und durch ihr Tragen oder Mitführen in Wechselwirkung mit der Berechtigungsvorrichtung eine oder mehrere Tätigkeiten mit oder an der medizinischen Vorrichtung und/oder einer oder mehreren Bewegungen der medizinischen Kraft (U) im Umfeld der medizinischen Vorrichtung erlauben oder verbieten kann, auch eine oder mehrere Identifikationsvorrichtungen umfassen, welche zum Tragen z. B. durch Patienten oder Besucher vorgesehen ist, und welche vorzugsweise kein Berechtigung verleihen, sondern vom erfindungsgemäßen Behandlungssystem in dessen Funktion einbezogen werden, um den Aufenthalt oder Weg ihres Trägers jetzt oder in der Vergangenheit z. B. im Umfeld nachvollziehen und in Bezug zu Aufenthalten oder Wegen des Trägers weitere Identifikationsvorrichtungen (z. B. Marker, Markierungssysteme, usw.) zu setzen, um hieraus Ergebnisse zu formulieren, um z. B. Berechtigungen zu widerrufen oder zu ändern. Letzterem kann ein Hinweis an den Träger, dessen Berechtigung vor einem möglichen Widerruf steht, ergehen. Der Hinweis kann eine Information sein, er kann eine Handlungsanweisung umfassen. Die Handlungsanweisung kann, sofern ihr nachgekommen wurde, optional dazu führen, dass der Widerruf der Berechtigung nicht eintritt.

In manchen Ausführungsformen ist ein weiterer Auslöser zum Widerrufen von Berechtigungen das Erfassen einer nicht identifizierten Person durch das medizinische Behandlungssystem. Beispielsweise, wenn nach dem Erfassen durch eine Sensoranordnung die Person nicht mittels der Lesevorrichtung identifiziert werden kann. So kann beispielsweise einer medizinischen Kraft, die eine Zugangsberechtigung für einen Behandlungsraum und die sich darin befindenden Behandlungsvorrichtungen hat, aus Sicherheitsgründen der Zugriff auf die Funktionen der Blutbehandlungsvorrichtung verwehrt werden, wenn eine nicht zu identifizierende Person im Umfeld der Behandlungsvorrichtung erkannt wird, z. B. ein Besucher, den die medizinische Kraft vorher aufgrund ihrer Berechtigung in den Behandlungsraum mitgenommen hat.

In manchen Ausführungsformen ist oder umfasst die Identifizierungsvorrichtung eine (Chip- oder Magnet-)Karte, eine Erkennungsmarke (Badge), ein Armband, ein Implantat, Bekleidung (oder ist hiermit verbunden), eine Sehhilfe, beispielsweise eine Brille oder Kontaktlinsen, Schuhbestandteile oder -einlagen, Accessoires hierfür, grafische Drucke, beispielsweise als Aufkleber mit einem Code, aktive oder passive Transponder und/oder ein handgehaltenes Gerät, beispielsweise ein Smartphone, ein Piepser, ein PDA (*Personal Digital Assistant*), usw., um jeweils die Informationen hieraus zu erfassen.

Grafische Drucke können beispielsweise als Aufkleber, der z. B. einen Code (z. B. Barcode oder QR-Code) abbildet, realisiert sein. Diese Aufkleber können gut sichtbar an der Bekleidung, beispielsweise einem Kittel, befestigt werden. Armbänder mit solchen Codes sind ebenfalls von der vorliegenden Erfindung umfasst.

Alternativ oder ergänzend zu den oben genannten Identifizierungsmitteln können medizinische Kräfte der Klinik/Praxis durch eine Kombination von Kamera und WiFi-Scannern, WLAN oder anderen Vorrichtungen identifiziert werden, die geeignet sind, die Bewegung oder das Bewegungsmuster der medizinischen Kraft zu erfassen. Hierzu können vorzugsweise einige, manche oder alle zugangsbeschränkten Bereiche der Klinik oder der Praxis und/oder die Wege dazwischen durch ein System von Kameras und WiFi-Scannern abgedeckt sein, das seinerseits z. B. mit dem Netzwerk des medizinischen Behandlungssystem, der Klinik oder der Praxis verbunden ist. Das Bewegungsmuster der passierenden medizinischen Kraft kann erfasst und z. B. an den Server (Lesevorrichtung) übermittelt werden, wo die charakteristischen Signaturen der Bewegung, beispielsweise das Gangmuster, mit hinterlegten Signaturen, die der medizinischen Kraft eindeutig zuzuordnen sind, verglichen werden können, womit eine Identifizierung der betreffenden medizinischen Kraft möglich sein kann. Der medizinischen Kraft werden die ihrer Identität entsprechenden Berechtigungen erteilt, und nachfolgend kann sie im Rahmen der ihr erteilten Berechtigungen frei agieren.

WiFi-Scanner können beispielsweise Identifizierungsvorrichtungen erfassen, welche in einem zugehörigen Drahtlosnetzwerk (WLAN) angemeldet sind.

In manchen Ausführungsformen ist die Identifizierungsvorrichtung in eine Brille integriert oder mit dieser verbunden, die außerdem einen Anzeigeabschnitt, mit welchem dem Träger Informationen zur Verfügung gestellt werden können, eine Kamera und Kommunikationsmittel (z. B. WiFi) umfasst. Diese Brille kann an die medizinischen Kräfte beispielsweise bei dem Betreten der Klinik/Praxis ausgegeben werden. Das Identifizieren der medizinischen Kräfte kann dann mittels der Kamera, beispielweise mittels Augenscan oder Irisscan, beim Aufsetzen der Brille erfolgen und zusätzlich an sensiblen Orten während des Arbeitsablaufs oder vor deren Betreten.

Berührungsfreie Mittel zur Steuerung einer medizinischen Vorrichtung, beispielsweise der Behandlungsvorrichtung, können beispielsweise mittels sogenannter "*head-up*"-Displays, beispielsweise integriert in den oben genannten Anzeigeabschnitt der Brille, realisiert werden. So kann dort beispielsweise ein virtuelles Touchpad eingeblendet werden, dessen Bedienung mittels Gestensteuerung oder Augenbewegung, welche beispielsweise von der Brille beispielsweise über die Kamera erfasst werden kann, erfolgen kann.

In manchen Ausführungsformen weist das medizinische Behandlungssystem weiter ein Ausgabe- und/oder Abgabeterminal auf. Dieses ist programmiert zum Erstellen, Beschreiben und/oder Auslesen einer Identifizierungsvorrichtung, welche zu ihrem Mitführen durch die medizinische Kraft während des Aufenthalts in der Klinik oder Praxis vorgesehen ist.

Das Ausgabe- und/oder Abgabeterminal ist vorzugweise programmiert, um die von ihm erstellte oder beschriebene Identifizierungsvorrichtung mit Informationen zu versehen, welche ihrem Träger vorzugsweise zeitlich und/oder räumlich beschränkt eine oder mehrere Tätigkeiten mit oder an der medizinischen Vorrichtung und/oder eine oder mehrere Bewegungen im Umfeld der medizinischen Vorrichtung erlauben oder verbieten.

So kann vorgesehen sein, dass für eine Pflegekraft, welche als medizinische Kraft morgens zum Dienst erscheint, am Ausgabe- und/oder Abgabeterminal eine Identifizierungsvorrichtung ausgedruckt wird, welche sie sich z. B. als QR-Code-Aufkleber gut sichtbar an den Kittel heftet. Die dem QR-Code von der Lesevorrichtung oder der Berechtigungsvorrichtung entnehmbaren Informationen können die Pflegekraft berechtigen, sich an bestimmte Orte der Klinik hinzubewegen (weil z. B. sich Türen auf dem Weg dorthin für diese medizinische Kraft aufgrund der von ihr getragenen Identifizierungsvorrichtung öffnen (lassen)), an andere, etwa wegen einer Infektion, welche die Pflegekraft kürzlich durchgemacht hat, (noch) nicht, oder erst in einigen Tagen wieder.

Ebenso kann z. B. vorgesehen sein, dass für einen Techniker, der zum Warten einer oder mehrerer Behandlungsvorrichtung(en) in der Klinik erscheint, an der Ausgabe- und/oder Abgabeterminal eine Identifizierungsvorrichtung ausgestellt wird (als QR-Code, als Chipkarte, usw.), welcher ihn für den begonnenen Arbeitstag, aber eben nicht länger, berechtigt, sich zu den fraglichen Behandlungsvorrichtungen zu bewegen. Türen und dergleichen auf dem Weg dorthin können für ihn, veranlasst durch die Berechtigungsvorrichtung, geöffnet werden.

Bei späterer Abgabe oder Rückgabe von Identifizierungsvorrichtungen durch die medizinische Kraft am Ausgabe- und/oder Abgabeterminal, z. B. bei Verlassen der Klinik/Praxis, kann die Identifizierungsvorrichtung bei entsprechender Programmierung automatisch ausgelesen werden.

So kann vorgesehen sein, dass das Ausgabe- und/oder Abgabeterminal ermittelt, wie viele Stunden die Pflegekraft an diesem Tag gearbeitet hat, oder wie viel Zeit der Techniker im Haus verbracht hat. Ebenso kann ermittelt werden, welche Patienten die medizinische Kraft betreut hat und an welchen Behandlungsvorrichtungen der Techniker tätig gewesen ist, was über den Tag hinweg ebenfalls in der Identifizierungsvorrichtung hinterlegt worden sein kann. Letzteres ist insbesondere beim Nachverfolgen von Infektionsketten oder beim Erstellen von Kostenrechnungen nach Leistungserbringung von Vorteil.

Das Ausgabe- und/oder Abgabeterminal kann programmiert sein, die Identifizierungsvorrichtung nach ihrer Abgabe oder ihrem Auslesen ungültig zu machen oder zu entwerten.

In einigen Ausführungsformen ist oder umfasst die Behandlungsvorrichtung eine Infusionspumpe, ein Beatmungsgerät, eine extrakorporale Blutbehandlungsvorrichtung, insbesondere eine Dialysevorrichtung, Hämodialysevorrichtung, Hämofiltrationsvorrichtung oder Hämodiafiltrationsvorrichtung, insbesondere eine Vorrichtung für die akute, die chronische Nierenersatztherapie oder für die kontinuierliche Nierenersatztherapie (CRRT = *continuous renal replacement therapy*).

In manchen Ausführungsformen ist die Diagnosevorrichtung ein Röntgengerät, ein Computertomograph, ein Magnetresonanztomograph (MRT), ein Ultraschallgerät, ein EKG-Gerät, ein Gerät für die Endoskopie, ein Gerät zur Impedanzmessung, ein Gerät zur Thermografie, eine Gammakamera und/oder eine andere Vorrichtung für die Diagnose, insbesondere mittels bildgebender Verfahren, oder umfasst wenigstens eines dieser Geräte oder eine dieser Vorrichtungen.

In einigen Ausführungsformen ist die weitere medizinische Klinikvorrichtung ein Bildschirm zur medizinischen Überwachung, ein medizinischer Kühlschrank oder ein medizinisches Kühlfach, eine Lagereinrichtung zum Lagern von medizinischen Einwegartikeln und/oder medizinischen Instrumenten oder umfasst wenigstens eines dieser Geräte oder eine dieser Vorrichtungen. Die Lagereinrichtung kann beispielsweise das Mobiliar des Lagers oder Teile hiervon umfassen, d. h. die Türen beispielsweise eines Schranks.

Des Weiteren fällt unter die medizinischen Klinikvorrichtungen in manchen Ausführungsformen beispielsweise eine Zugangstür, etwa zu einer Station, einer Infektionsabteilung, der Klinik/Praxis, einem Behandlungsraum, einem Patientenzimmer, einem Lagerraum, einem Sozialraum, einer Umkleide, einem Schließfächerbereich, usw.

In manchen Ausführungsformen sind die Tätigkeiten Schritte oder Handlungen zum Betätigen, Bedienen oder Verwenden der medizinischen Vorrichtung aus der Gruppe von Elementen oder umfassen solche Schritte oder Handlungen, insbesondere für einen bestimmungsgemäßen Gebrauch der medizinischen Vorrichtung, z. B. bei oder zu ihrer Verwendung während der Diagnose oder bei der Behandlung des Patienten.

Zu den Tätigkeiten im Sinne der Erfindung zählen ferner beispielweise ein Starten oder Anhalten von Behandlungssitzungen, das Reagieren auf Alarme, das Betrachten oder Auswerten von mittels der medizinischen Vorrichtung erhobenen Diagnosen oder Untersuchungsergebnissen und dergleichen. Alternativ oder ergänzend umfassen die Tätigkeiten im Sinne der Erfindung optional solche, welche dem Vorbereiten der medizinischen Vorrichtung für die Untersuchung, für die Behandlung usw. oder zum Vorbereiten des Patienten, beispielweise für eine Behandlungssitzung, dienen, wie z. B. das Veranlassen eines Kanülierungsroboters, eine Kanülierung vorzunehmen.

In einigen Ausführungsformen sind oder umfassen die Bewegungen ein Sich-Annähern der medizinischen Kraft an die medizinische Vorrichtung beispielsweise indem die medizinische Kraft das Umfeld der medizinischen Vorrichtung betritt, oder ein Sich-Entfernen, beispielsweise ein Verlassen des Umfelds der medizinischen Vorrichtung, ein Passieren, beispielsweise eines Gangs, eines Treppenhauses oder dergleichen, ein Betreten oder ein Verlassen, beispielweise eines Raumes, oder ein Aufenthalt im Umfeld der medizinischen Vorrichtung, jeweils durch die medizinische Kraft.

In manchen Ausführungsformen ist oder umfasst das Umfeld eine vorbestimmte und/oder überwachte Fläche oder ein vorbestimmter und/oder überwachter Raum der Klinik/Praxis.

Insbesondere ist dies ein Bereich, der nur mit Berechtigung, z. B. nur von der medizinischen Fachkraft, jeweils ausgewählten Technikern, ausgewählten Patienten oder ausgewählten Besuchern usw., betreten werden darf.

Alternativ oder ergänzend ist auch das Überwachen eines Eingangsbereichs der Klinik oder Praxis oder jedes anderen öffentlich oder auch für Personen ohne besondere Berechtigung zugänglichen Bereichs, beispielsweise hierin als Übergangsbereiche bezeichnete, Gänge oder Treppenhäuser, von der vorliegenden Erfindung umfasst.

Diese Bereiche gehören in manchen Ausführungsformen somit ebenfalls zum Umfeld.

Ferner kann ein Bereich, in welchem sich durch die Anwesenheit der medizinischen Kraft zu bestimmten Zeiten der Arbeitsbeginn und/oder das Arbeitsende definieren lassen, beispielsweise eine Umkleide, in manchen Ausführungsformen überwacht und somit zum Umfeld gerechnet werden.

Vorzugsweise werden wenigstens ein Behandlungsraum, wenigstens ein Überwachungsraum, wenigstens ein Raum, in welchem die medizinische Vorrichtung aufgestellt ist, wenigstens ein Raum mit Zugangsbeschränkung, beispielsweise Lager, insbesondere Medikamentenlager, Küche, Vorbereitung, usw., und/oder der Übergangsbereich, d. h. der Weg über einen Gang, Treppenhaus oder dergleichen, von einem zum anderen der vorgenannten Räume überwacht und sind somit in manchen Ausführungsformen das Umfeld oder von diesem umfasst.

In einigen Ausführungsformen weist das erfindungsgemäße medizinische Behandlungssystem eine Schreibvorrichtung auf, welche programmiert ist, um - vorzugsweise kontaktlos - Daten oder Informationen auf die Identifizierungsvorrichtung zu schreiben oder zu überschreiben. Beim Schreiben oder Überschreiben auf die Identifizierungsvorrichtung werden Informationen erstellt, welche von der Lesevorrichtung lesbar sind.

In manchen Ausführungsformen weist das erfindungsgemäße medizinische Behandlungssystem eine Schreibvorrichtung auf, welche zum - vorzugsweise kontaktlosen - Schreiben oder Überschreiben von Daten oder Informationen auf die/der Berechtigungsvorrichtung programmiert ist. Beim Schreiben oder Überschreiben werden Informationen erstellt, welche von der Lesevorrichtung lesbar sind.

In manchen Ausführungsformen ist die identifizierte medizinische Kraft automatisch berechtigt, wenn sie zu einer ersten Tätigkeit A berechtigt war, auch eine weitere Tätigkeit B, die auf die Tätigkeit A folgt, vorzunehmen. Beispielsweise kann es möglich sein, dass wenn die medizinische Kraft berechtigt war, einen Behandlungsraum zu betreten, sie automatisch berechtigt ist oder vom medizinischen Behandlungssystem berechtigt wird, auch Einstellungen z. B. an einer Behandlungsvorrichtung, vorzunehmen. Ebenso können andere Personen von Schritt B und evtl. nachfolgenden Schritten ausgeschlossen aktiv werden, indem ihnen die Berechtigung nicht erteilt oder entzogen wird, etwa den Schritt B und weitere durchzuführen.

In einigen Ausführungsformen können bei Vorliegen eines Notfalls die Bedingungen für das Erteilen einer Berechtigung geändert werden und/oder Anforderungen für die Identifizierung gesenkt werden. Beispielsweise kann in einem Alarmfall jede medizinische Kraft, die den Behandlungsraum betreten darf und/oder durfte, berechtigt sein oder werden, auch Steuereingaben an der Behandlungsvorrichtung zu tätigen. Optional können nur Eingaben an der Behandlungsvorrichtung autorisiert werden, welche im Zusammenhang mit dem Alarmfall stehen. Dabei kann die Anwesenheit von wenigstens einer berechtigten Person im Behandlungsraum oder im Umfeld der Behandlungsvorrichtung als Voraussetzung ausreichen. Siehe hierzu auch das nachfolgend beschriebene Beispiel (3).

Die Identifizierungsinformationen können in einigen Ausführungsformen mittels Daten-Vorverarbeitung dergestalt anonymisiert werden, dass bestimmte Vorgaben, beispielsweise des Datenschutzes oder der technischen Datenstrom- oder (virtuellen) Speicherlimitierung, eingehalten werden können. Hierzu kann während der Daten-Vorverarbeitung bereits eine Datenreduzierung stattfinden, beispielsweise indem ein Bild eines Gesichtes auf ein Gesichtsmuster oder ein Bild eines Fingers auf die Positionen von Fingerabdruckscheitelpunkten reduziert wird. Die verbleibenden Daten sind ausreichend für die Identifizierung der medizinischen Kraft. Die Daten-Vorverarbeitung kann bereits in der Elektronik der betreffenden Sensoren noch vor Weitergabe an die Lesevorrichtung erfolgen. Hierdurch kann durch die Datenreduzierung zwar weiterhin eine Identifizierung durch beispielsweise die Lesevorrichtung erfolgen, beim Auslesen der Identifizierungsinformationen durch einen Dritten jedoch keine Rückschlüsse auf die sich identifizierende Kraft gezogen werden.

Alle medizinischen Vorrichtungen, Behandlungsvorrichtungen, Diagnosevorrichtungen und weitere medizinische Vorrichtungen können innerhalb des definierten Umfelds untereinander in manchen Ausführungsformen mit wenigstens einer weiteren der vorgenannten Vorrichtung in zweiseitiger Datenkommunikation stehen. Ebenso können sie mittels eines Netzwerks in zweiseitiger Datenkommunikation mit Zentralrechnern und/oder Daten(-bank-)Servern innerhalb oder außerhalb des definierten Umfelds, insbesondere innerhalb oder außerhalb der Klinik, stehen. Das Netzwerk kann vollständig oder in Teilen kabellos verbunden sein, beispielsweise mittels WLAN, WiFi, Bluetooth.

Jede erfasste oder gespeicherte Information, seien es Video-, Audio- oder sonstige Daten, kann automatisch in ein Format überführt werden, das eine menschliche Nutzung, Verarbeitung oder Manipulation ausschließt. Insbesondere kann hierdurch eine weitergehende Überwachung von Personen, über deren Identifizierung und Autorisierung hinaus, vorzugsweise ausgeschlossen werden.

Folgende Beispiele sind ebenfalls von der vorliegenden Erfindung umfasst:
(1) Eine Person, die nicht dem Klink/Praxis-Personal angehört, beispielsweise der Besucher eines Patienten, versucht, einen bestimmten Raum zu betreten. Da das medizinische Behandlungssystem diese Person nicht identifizieren (und vermutlich noch weniger eine entsprechende Berechtigung für sie ermitteln) konnte, bleibt die gesicherte Tür zu diesem Raum geschlossen. Eine medizinische Kraft, die sich in der Nähe aufhält, kann den Besucher möglicherweise als solchen bestätigen und wird z. B. nach Einlesen ihrer Identifikationsvorrichtung durch die Lesevorrichtung (z. B. verbunden mit einer Kamera vor der Tür) und von der Berechtigungsvorrichtung als autorisiert erkannt, den Raum zu betreten. Sie kann dem Besucher die Türe öffnen oder zusammen mit ihm den Raum betreten. Sollte sich der Besucher an einer in diesem Raum vorhandenen Behandlungsvorrichtung bewusst oder unbewusst zu schaffen machen wollen, so ist ihm dies in manchen Ausführungsformen nicht möglich, da es zum Bedienen der Behandlungsvorrichtung dieses Beispiels einer weiteren Berechtigung bedarf, die für den Besucher wiederum nicht ermittelt werden konnte da der Besucher nicht als autorisiert identifiziert worden ist.
(2) Eine medizinische Kraft, die zum Klinik-/Praxis-Personal gehört, betritt die Klinik, ohne vom erfindungsgemäßen medizinischen Behandlungssystem identifiziert oder autorisiert zu werden. Die Tür zur Personalumkleide verlangt jedoch die Eingabe einer Benutzernamen/Passwort-Kombination. Selbst wenn die medizinische Kraft ihr Passwort vergessen hätte, könnte sie an dieser Stelle mittels eines anderen Identifizierungsverfahrens identifiziert werden, beispielsweise, indem sie einige Sätze laut vorliest. Ihre Stimme wird von einem Mikrofon erfasst, und sie wird hierüber von der Lesevorrichtung identifiziert. Die medizinische Kraft kann nun ganz normal ihren Arbeitstag beginnen, da sie mittels des medizinischen Behandlungssystems, insbesondere dessen Berechtigungsvorrichtung, die ihr zustehenden Berechtigungen erhält. Beispielsweise wird ihr Schichtbeginn automatisch erfasst, beispielsweise in einem Arbeitszeiterfassungssystem, ein Aufkleber, der einen personalspezifischen QR-Code enthält, wird gedruckt und an die medizinische Kraft ausgegeben. Die medizinische Kraft bringt diesen Aufkleber gut sichtbar an ihrer Kleidung, beispielsweise einem Kittel, an, und der Code wird anschließend mittels Sensoranordnungen mit grafischer Erkennung, beispielsweise mittels Kameras, an den Türen und Behandlungsvorrichtungen identifiziert und entsprechend autorisiert. Nach der Schicht nimmt die medizinische Kraft den Aufkleber wieder ab, beispielsweise vom Kittel, und scannt ihn, beispielsweise in der Umkleide, um ihn zu entwerten. Hiermit kann in manchen Ausführungsformen auch die Erfassung des entsprechenden Schichtendes, beispielsweise in einem Arbeitszeiterfassungssystem, verbunden sein.
(3) Bei Auftreten einer Notfallsituation, z. B. in einem Behandlungsraum, können die Anforderungen an die Identifizierung in manchen Ausführungsformen automatisch gesenkt werden.

Hat beispielsweise die medizinische Kraft ihre Identifizierungsvorrichtung liegen lassen und hat keine Zeit zu suchen, weil sie zu dem Behandlungsraum mit dem Notfall rennt, werden ihre Bewegungen z. B. von Kameras und WiFi-Sensoren auf dem Weg dorthin erfasst und ihre Identität von der Lesevorrichtung identifiziert. Die Berechtigungsvorrichtung kann dann der Tür zum Behandlungsraum bereits aufgrund dieser Identifizierung erlauben, von der medizinischen Kraft geöffnet zu werden, und die Behandlungsvorrichtung, an der der Notfall eingetreten ist, kann beim Betreten des Raums bereits für Eingaben entsperrt werden. Verwechselt die medizinische Kraft in der vorliegenden, stressigen Situation jedoch die Kontrollbefehle der Behandlungsvorrichtung und erteilt sie der Behandlungsvorrichtung beispielsweise den Befehl, herunterzufahren, so verweigert die Behandlungsvorrichtung dies, da die medizinischen Kraft hierzu nicht wiederum nicht autorisiert war und ist. Die medizinische Kraft bekommt erneut die Gelegenheit, einen - anderen - Befehl einzugeben, der geeignet ist, die Notfallsituation zu beheben.

Manche oder alle erfindungsgemäßen Ausführungsformen können einen, mehrere oder alle der oben und/oder im Folgenden genannten Vorteile aufweisen.

Mittels der vorliegenden Erfindung kann vorteilhafterweise unbefugter Zugang zu medizinischen Vorrichtungen verhindert werden, was der Sicherheit im Klinikablauf dienen (Diebstahl, Manipulation, Verunreinigung, usw.) und mittelbar die Sicherheit des Patienten erhöhen kann.

Mittels der vorliegenden Erfindung kann vorteilhafterweise unbefugter Zugang zu Patienten verhindert werden, was ebenfalls der Sicherheit des Patienten zugutekommen kann, etwa weil der Patient auf einer Isolierstation liegt und z. B. Besucher oder selbst eine nicht entsprechend gekleidete medizinische Kraft keinen Zugang zum Patienten haben soll.

Mittels der vorliegenden Erfindung kann vorteilhafterweise verhindert werden, dass unbefugt (mangels Berechtigung) medizinische Vorrichtungen der Klinik oder Praxis gewollt oder ungewollt (z. B. durch Ablegen der Handtasche oder des mitgebrachten Blumenstraußes auf Schaltflächen einer Behandlungsvorrichtung) bedient werden und/oder Behandlungsschritte veranlasst werden können. Dies kann der Sicherheit des Patienten dienen.

Durch Verwenden der vorliegenden Erfindung kann der zur Identifizierung klinischen Personals erforderliche Aufwand bei der Betätigung von Identifikationseinrichtungen, beispielsweise an oder integriert in medizinische(n) Geräten, erheblich reduziert werden. Dies kann helfen, den Arbeitsablauf zu optimieren sowie Zeit und Geld einzusparen.

Durch Verwenden der vorliegenden Erfindung im Zusammenhang mit erforderlichen Hygienemaßnahmen, kann die medizinische Sicherheit im Klinikablauf erhöht werden, insbesondere durch ein Vor-Berechtigen der medizinischen Kraft, was das Betätigen von mehr als einer Identifikationseinrichtungen pro Tag entbehrlich werden lassen kann.

Ein weiterer Vorteil der vorliegenden Erfindung kann in der räumlichen und zeitlichen Trennung zwischen der Identifizierung von medizinischen Kräften und der Bedienung bzw. Benutzung von medizinischen Vorrichtungen liegen. Dies kann helfen, den Arbeitsablauf im Umfeld zu optimieren und damit Zeit und Geld einzusparen.

Durch Verwendung der vorliegenden Erfindung können vorteilhaft geläufige Systeme zum Identifizieren von medizinischen Kräften ersetzt werden. So können Schlüssel zum Öffnen von Kliniktüren oder andere traditionelle Mittel, die leicht verloren oder verlegt werden können, durch sie ersetzt werden, was vorteilhaft die Sicherheit im Arbeitsablauf der medizinischen Kraft erhöhen aber auch deren Vereinfachung bedeuten kann. Das Verbinden der - wie auch immer ausgestalteten - Identifizierungsvorrichtung mit oder an medizinischer Bekleidung (Handschuhe, Kittel, Brillen, Masken, Schuhen) bietet ebenfalls eine Sicherung gegen Verlust, da solche Gegenstände regelmäßig in der Klinik verbleiben und dort z. B. nach Arbeitsende von der medizinischen Kraft weggeschlossen werden können.

Im Folgenden wird die vorliegende Erfindung unter Bezugnahme auf die beigefügte Zeichnung rein exemplarisch beschrieben. In ihr bezeichnen selbe Bezugszeichen gleiche oder selbe Komponenten. In den Figuren der Zeichnungen gilt:
- **Fig. 1**: zeigt in vereinfachter Darstellung ein medizinisches Behandlungssystem in einer ersten Ausführungsform;
- **Fig. 2**: zeigt in vereinfachter Darstellung eine Behandlungsvorrichtung und eine weitere Klinikvorrichtung mit ihrem jeweiligen Umfeld als Teil eines medizinischen Behandlungssystems in einer zweiten Ausführungsform;
- **Fig. 3**: zeigt in stark vereinfachter Darstellung eine Behandlungsvorrichtung in einem Behandlungsraum und deutet die Überwachung des Zugangs zu diesem Behandlungsraum mittels einer Sensoranordnung an;
- **Fig. 4**: zeigt in vereinfachter Darstellung ein erfindungsgemäßes medizinisches Behandlungssystem in einer weiteren Ausführungsform;
- **Fig. 5**: zeigt einen Ablaufplan eines Beispiels für die Verwendung des erfindungsgemäßen medizinischen Behandlungssystems in einer exemplarischen Ausführungsform; und
- **Fig. 6**: zeigt einen Ablaufplan eines Beispiels für die Verwendung des erfindungsgemäßen medizinischen Behandlungssystems in einer weiteren exemplarischen Ausführungsform.

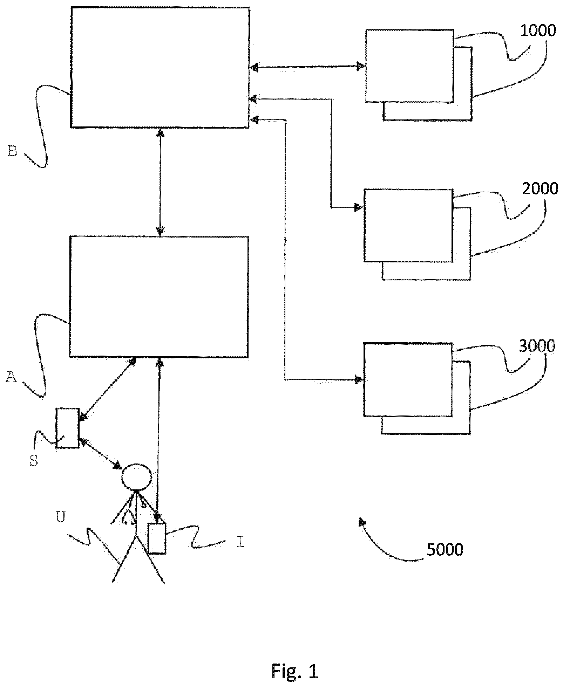

**Fig. 1** zeigt in vereinfachter Darstellung ein medizinisches Behandlungssystem 5000 in einer ersten Ausführungsform.

Es umfasst eine Lesevorrichtung A, die als Ein- oder Auslesevorrichtung ausgestaltet sein kann. Sie ist programmiert, um Daten oder Informationen (im Folgenden auch kurz: Informationen) zu erfassen, die einer Identifizierung einer medizinischen Kraft U und/oder dem Ermitteln deren Autorisierung für bestimmte Bewegungen oder Tätigkeiten dienen kann. Dieses Erfassen erfolgt vorzugsweise kontaktlos.

Die medizinische Kraft U kann ein Behandelnder (Arzt/Ärztin) und/oder eine Pflegekraft (Pfleger/Schwester) sein oder dem erweiterten Klinikpersonal angehören, beispielsweise ein (System-)Techniker, ein Hausmeister, eine Reinigungskraft usw. sein.

Die Lesevorrichtung A ist dazu programmiert, die Identität der medizinischen Kraft U zu erkennen, zu ermitteln oder festzustellen. Sie kann zu diesem Zweck beispielsweise auf Daten und/oder Informationen zugreifen, welche von einer Identifizierungsvorrichtung I ausgehen bzw. bereitgehalten werden, die die medizinische Kraft U optional mit sich trägt.

Die Identifizierungsvorrichtung I kann beispielweise eine Chipkarte, eine Magnetkarte, ein Armband, ein Implantat, ein Badge und/oder ein handgehaltenes Gerät sein. Die Identifizierungsvorrichtung I kann in die Bekleidung, eine Sehhilfe oder dergleichen der medizinischen Kraft U integriert sein.

Die Lesevorrichtung A kann optional ergänzend oder alternativ programmiert sein, um z. B. mittels einer oder mehrerer Sensoranordnungen S beispielsweise biometrische Informationen, persönliche Bewegungsmuster zu erkennen und/oder persönliches Wissen abfragen oder zu erfassen und ggf. in verarbeitbare oder übermittelbare Informationen zu wandeln. Derartige Informationen können für den Fachmann erkennbar von der Lesevorrichtung A erfasst oder generiert werden, ohne dass die medizinische Kraft U hierzu eine Identifizierungsvorrichtung I, wie sie in Fig. 1 als Option gezeigt ist, mit sich führen müsste. Informationen, die von einer oder mehreren Sensoranordnungen S erfasst wurden, können von diesen an die Lesevorrichtung A kabellos, kabelgebunden oder auf andere Weise übermittelt werden bzw. von dort mittels der Lesevorrichtung A ausgelesen werden. Alternativ kann eine beliebige der hierin genannten Sensoranordnungen S Teil der Lesevorrichtung A oder mit dieser identisch sein.

Die Lesevorrichtung A, oder eine andere Vorrichtung des Behandlungssystems 5000, ist optional ferner dazu programmiert, die von ihr - gleich auf welchem Wege - erfassten Informationen mit, beispielsweise in ihr oder in einem mit ihr verbundenen Speichermedium, beispielsweise einer Datenbank (nicht in Fig. 1 gezeigt), hinterlegten Informationen zu vergleichen.

Die Lesevorrichtung A ist dazu programmiert, die von ihr erfassten und ggf. verarbeiteten Informationen für weitere Vorrichtungen, beispielsweise für die Berechtigungsvorrichtung B, bereitzustellen und/oder an diese zu übermitteln.

Rechts in Fig. 1 sind je zwei (oder, unabhängig voneinander mehr oder weniger als zwei, z. B., keine, eine, zwei oder mehr) Behandlungsvorrichtungen 1000, Diagnosevorrichtungen 2000 und Klinikvorrichtungen 3000 gezeigt, die im Folgenden zusammen als Gruppe bezeichnet werden. Die in der Fig. 1 gewählte Anzahl von zwei Behandlungsvorrichtungen 1000, zwei Diagnosevorrichtungen 2000 und zwei weiteren Klinikvorrichtungen 3000 ist somit rein beispielhaft zu verstehen.

Die Berechtigungsvorrichtung B ist programmiert, um, basierend auf den von der Lesevorrichtung A erfassten Informationen, Bewegungen und/oder Tätigkeiten der medizinischen Kraft U im Zusammenhang mit oder an einem der Elemente der Gruppe (also wenigstens einer bestimmten Behandlungsvorrichtung 1000, Diagnosevorrichtung 2000 oder weiteren Klinikvorrichtung 3000) entweder zu erlauben oder zu verbieten.

Das Erlauben oder Verbieten erfolgt mittels der Berechtigungsvorrichtung B, die gemeinsam mit der Lesevorrichtung A, als Teil von ihr oder unabhängig von ihr vorgesehen sein kann.

Auf der Identifizierungsvorrichtung I kann eine Anzeige oder eine Benachrichtigung bezüglich der Berechtigung zur Kenntnisnahme durch die medizinische Kraft U vorgesehen sein. Weiter kann vorgesehen sein, der medizinischen Kraft U auf diese Weise mitzuteilen, weshalb ihr z. B. der Zugang zu einem bestimmten Raum untersagt wird, etwa in Form von Textnachrichten wie "Fehlende Schutzausrüstung" oder dergleichen.

Das Erlauben kann zum Beispiel ein Entriegeln einer ansonsten verschlossenen Tür als Beispiel für eine weitere Klinikvorrichtung 3000 sein, so dass die als berechtigt erkannte Kraft U die Tür öffnen kann, die Tür für die Kraft U automatisch geöffnet wird, usw. Das Erlauben kann aber die Möglichkeit für die medizinische Kraft U sein, eine Behandlung des Patienten mittels der Behandlungsvorrichtung 1000 zu starten, oder bestimmte Behandlungsoptionen auszuwählen, für welche besondere Berechtigungen erforderlich sind, die vorzugsweise überprüft werden sollten. Für Diagnosevorrichtungen 2000 kann Vergleichbares gelten.

Das Verbieten kann zum Beispiel ein Blockieren wenigstens einer Funktion des betreffenden Elements der oben genannten Gruppe, der Funktion insgesamt oder der Bedienbarkeit durch die konkrete medizinische Kraft U sein. So kann beispielsweise eine Eingabemöglichkeit an der Behandlungsvorrichtung 1000 oder der Diagnosevorrichtung 2000 blockiert sein, oder diese Vorrichtungen können bei Erkennen der (eingeschränkten) Berechtigung der Kraft U, was Behandlung oder Diagnose mittels dieser Vorrichtung betrifft, nurmehr ein eingeschränktes Spektrum ihrer grundsätzlich zur Verfügung stehenden Optionen anbieten. Ebenso kann das Verbieten durch Blockieren eines Zugangs (beispielsweise einer verschlossenen Tür) und/oder ähnliche Maßnahmen realisiert werden. Ein Verbieten kann aktiv oder passiv sein. So kann z. B. eine Tür (oder Funktion) bei Annähern durch die Kraft U, welche nicht berechtigt ist, sie zu durchschreiten, aktiv gesperrt werden. Alternativ kann bei Annähern der Kraft U eine Sperrung der Tür (oder der Funktion) einfach beibehalten werden, also nicht aufgehoben werden (passiv).

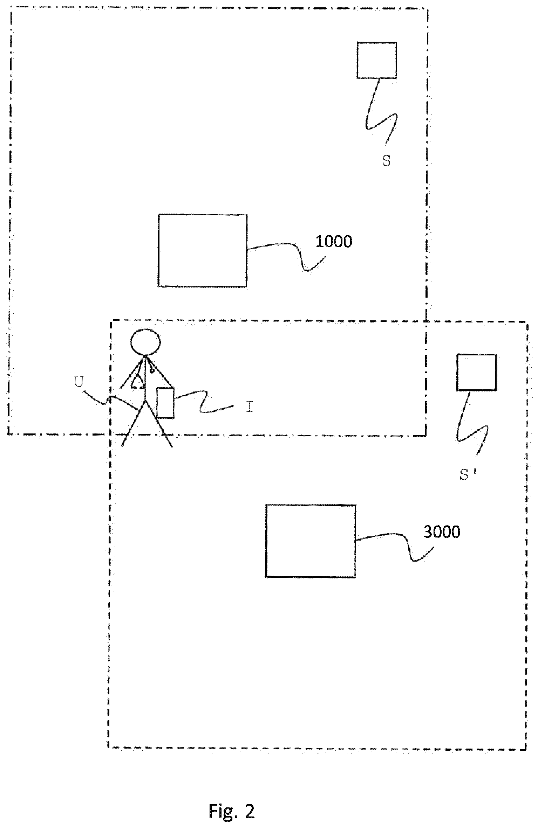

**Fig. 2** zeigt in vereinfachter Darstellung eine Behandlungsvorrichtung 1000 und eine weitere Klinikvorrichtung 3000 mit ihrem jeweiligen Umfeld als Teil eines medizinischen Behandlungssystems 5000 in einer zweiten Ausführungsform.

Das willkürlich festgelegte Umfeld der Behandlungsvorrichtung 1000 ist mit einer Strichpunktlinie angedeutet und wird mittels der Sensoranordnung S erfasst bzw. überwacht. Das Umfeld der weiteren Klinikvorrichtung 3000 ist mit einer Punktlinie angedeutet und wird mittels der Sensoranordnung S' erfasst bzw. überwacht.

Betritt eine medizinische Kraft U das Umfeld einer Vorrichtung wie der Behandlungsvorrichtung 1000 oder der weiteren Klinikvorrichtung 3000, so wird die medizinische Kraft U mittels der Lesevorrichtung A (siehe Fig. 1) identifiziert. Die hierzu erforderlichen Informationen werden entweder anhand von der jeweils zugeordneten Sensoranordnung S, S', beispielsweise einer Kamera, eines Scanners und/oder eines Radars, erfasst oder durch Auslesen von Informationen aus der Identifizierungsvorrichtung I, die die medizinische Kraft U optional mit sich trägt, ausgelesen.

Die Informationen zur Identität der medizinischen Kraft U werden anschließend mittels der Lesevorrichtung A an die Berechtigungsvorrichtung B übermittelt, alternativ liest die Berechtigungsvorrichtung B die entsprechenden Informationen aus der Lesevorrichtung A aus (siehe Fig. 1). Die Berechtigungsvorrichtung B ermittelt nun für die im medizinischen Behandlungssystem 5000 vorhandenen medizinischen Vorrichtungen, hier exemplarisch die Behandlungsvorrichtung 1000 und die weitere Klinikvorrichtung 3000, welche Berechtigungen für die jeweilige Vorrichtung 1000, 3000 für die medizinische Kraft U vorgesehen sind, und erteilt z. B. entsprechende Erlaubnisse gegenüber den betroffenen medizinischen Vorrichtungen. Derartige Berechtigungen können z. B. in einer Speichervorrichtung des medizinischen Behandlungssystem 5000 gespeichert sein.

Nähert sich die medizinische Kraft U beispielsweise der Behandlungsvorrichtung 1000, für die sie in diesem Beispiel keine Berechtigung haben soll, so kann die Berechtigungsvorrichtung B veranlassen, dass der medizinischen Kraft U der Zugang zu der oder der Zugriff auf die Behandlungsvorrichtung 1000 verboten (alternativ: eingeschränkt) oder nicht ermöglicht wird. Dies kann beispielsweise erfolgen, indem eine Tür zu einem Raum, in dem die Behandlungsvorrichtung 1000 steht, gar nicht erst geöffnet wird.

Als weiteres Beispiel sei hier das Sperren einer Eingabe an der Behandlungsvorrichtung 1000, z. B. durch Sperren eines Bildschirms, genannt. Die eingeschränkte Funktionsfähigkeit der Behandlungsvorrichtung 1000 ist in einem solchen Fall ebenfalls von der vorliegenden Erfindung umfasst, beispielsweise, indem auf dem Bildschirm der Behandlungsvorrichtung 1000 nur Informationen angezeigt und somit abzulesen sind, aber keine Eingabe, beispielsweise zur Änderung von Behandlungsparametern, durch die medizinische Kraft U ermöglicht wird. Beispielsweise kann eine Touchscreen-Funktion abgeschaltet sein oder bleiben, sollte die medizinische Kraft U nicht im erforderlichen Maße berechtigt sein.

Es kann in bestimmten, insbesondere in der vorliegenden, Ausführungsform der Fall sein, dass die medizinische Kraft U zwar keine Berechtigung für die Behandlungsvorrichtung 1000, aber sehr wohl Zugang zu einer weiteren Klinikvorrichtung 3000, beispielsweise einem Medikamentenschrank, der im selben Raum wie die Behandlungsvorrichtung 1000 steht, haben soll. Die Berechtigungsvorrichtung B wird der medizinischen Kraft U also den Zugang zu der weiteren Klinikvorrichtung 3000 erlauben, wenn die medizinische Kraft U im Umfeld der weiteren Klinikvorrichtung 3000 identifiziert wurde und sich ihr nähert. Das Erlauben kann ein Freischalten einer Türverriegelung zum Raum, in welchem die weitere Klinikvorrichtung 3000 steht, und/oder ein Entriegeln der Tür des Medikamentenschranks sein oder umfassen.

Einer medizinischen Kraft U, die für keine dieser beiden Vorrichtungen 1000, 3000 die erforderliche Berechtigung hat, z. B. ein *Facility Manager,* wird nach ihrem Identifizieren mittels der Lesevorrichtung A weder zur Behandlungsvorrichtung 1000 noch zur weiteren Klinikvorrichtung 3000 Zugang erhalten. Analog wird einer medizinischen Kraft U, die z. B. für beide Vorrichtungen 1000, 3000 eine Berechtigung besitzt, der Zugang zu diesen mittels der Berechtigungsvorrichtung B gewährt werden.

Dies gilt analog für alle möglichen Kombinationen aus Behandlungsvorrichtungen 1000, Diagnosevorrichtungen 2000 und weiterer Klinikvorrichtungen 3000.

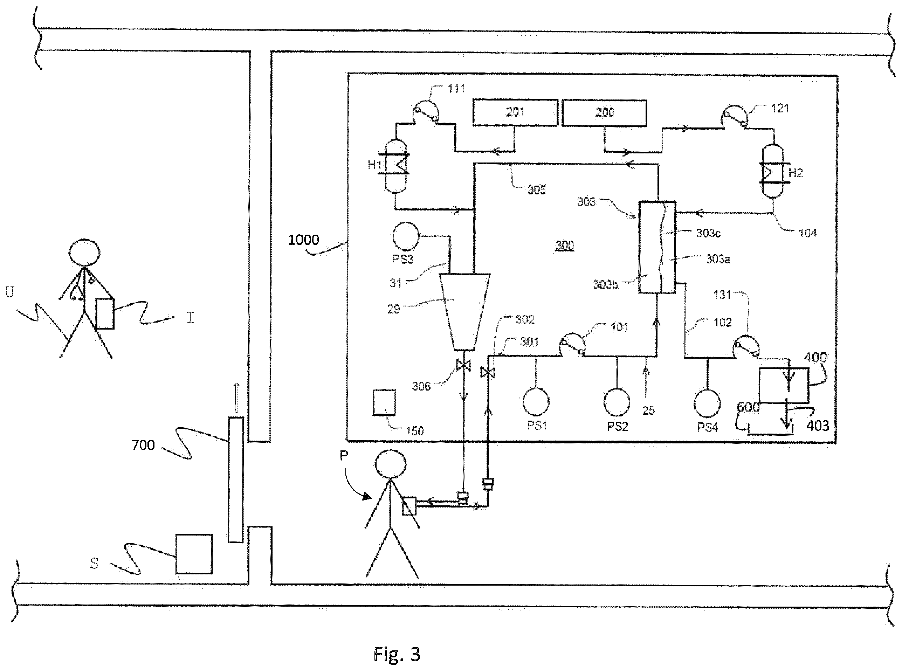

**Fig. 3** zeigt in stark vereinfachter Darstellung ein Verfahrensfließbild einer Behandlungsvorrichtung 1000, hier eine Blutbehandlungsvorrichtung, mit deren optionalen, extrakorporalen Blutkreislauf 300 ein Patient P verbunden ist. Die Behandlungsvorrichtung befindet sich in einem Behandlungsraum (rechts in Fig. 3). Der Zugang zu diesem Behandlungsraum ist mit einer Tür 700 verschlossen. Der Vorraum (links in Fig. 3) wird mittels einer Sensoranordnung S überwacht und gilt in diesem Beispiel bereits als Umfeld der Behandlungsvorrichtung 1000.

Die Fig. 3 zeigt die Blutbehandlungsvorrichtung 1000, optional verbunden mit einem Abflussschlauchsystem zu einem optionalen Effluentbeutel 400.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a, durch welche im Gebrauch Dialysierflüssigkeit geführt wird, und eine Blutkammer 303b, durch welche im Gebrauch Blut geführt wird, auf. Die Dialysierkammer 303a und die Blutkammer 303b sind zumeist mittels einer semi-permeablen Membran 303c voneinander getrennt. Blut und Dialysierflüssigkeit werden zumeist im Gegenstromprinzip durch den Blutfilter 303 geleitet. Das Blut wird im Blutfilter 303 gereinigt.

Der extrakorporale Blutkreislauf 300 weist ferner wenigstens eine zweite Leitung 305, hier in Form eines venösen Leitungsabschnitts, auf.

Sowohl die erste Leitung 301 als auch die zweite Leitung 305 dienen zu ihrer Verbindung mit dem Gefäßsystem des Patienten P.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 3 nur durch einige ihrer Einrichtungen und schematisch repräsentierte Blutbehandlungsvorrichtung 1000 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200, beispielsweise einem Beutel, heraus und über eine optional vorhandene Beutelheizung H2 mit einem Heizbeutel, entlang der Dialysierflüssigkeitszulaufleitung 104 in die Dialysierflüssigkeitskammer 303a des Blutfilters 303 gepumpt. Die Dialysierflüssigkeit verlässt die Dialysierflüssigkeitskammer 303a über die Dialysatablaufleitung 102 als Dialysat, ggf. angereichert durch Filtrat, in Richtung des Effluentbeutels 400. Das Filtrat kann Wasser umfassen, das dem Blut im Blutfilter entzogen wurde. Dialysat und Filtrat werden vereinfacht als Effluent bezeichnet.

Das Effluent wird in der Praxis mittels einer Dialysatablaufleitung 102 unmittelbar verworfen oder, vor allem bei der Akutbehandlung, dem Effluentbeutel 400 zugeführt und darin zunächst aufbewahrt. Nach Beendigung der Blutbehandlung, oder in während der Blutbehandlung liegenden Beutelleerintervallen (Intervalle, in welchen der Effluentbeutel 400 geleert wird), wird das Effluent aus dem Effluentbeutel 400, mittels einer Ausgussleitung 403 z. B. in ein Waschbecken oder einen anders gestalteten Ausguss 600 verworfen.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Neben der vorgenannten Blutpumpe 101 und der Pumpe für Dialysierflüssigkeit 121 weist die in Fig. 1 gezeigte Anordnung ferner rein optional eine Reihe weiterer, jeweils optionaler Pumpen, nämlich die Pumpe 111 für Substituat und die Pumpe 131 für das Effluent auf.

Die optionale Pumpe 111 ist vorgesehen, um Substituat aus der optionalen Quelle 201, beispielsweise einem Beutel, heraus und über eine optional vorhandene Beutelheizung H1 mit einem Heizbeutel der zweiten Leitung 305 zuzuführen.

Die optionale Pumpe 131 ist vorgesehen, um Effluent aus dem Blutfilter 303 heraus zu pumpen und in Richtung Effluentbeutel 400 zu fördern.

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, stromauf einer Zugabestelle 25 für Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 und/oder einen weiteren Drucksensor PS3 aufweisen kann. Anschließend wird das Blut in den Patienten zurückgeführt (reinfundiert).

Eine nur angedeutete Steuer- oder Regelvorrichtung 150 kann dazu konfiguriert sein, die Blutbehandlungsvorrichtung 1000 zum Zwecke einer Blutbehandlungssitzung zu regeln oder zu steuern. Sie ist in diesem Beispiel Teil der Blutbehandlungsvorrichtung 1000. In manchen Ausführungsformen kann die Steuer- oder Regelvorrichtung 150 getrennt von der Blutbehandlungsvorrichtung 1000 vorliegen.

Die Steuer- oder Regelvorrichtung 150 kann mit jeder beliebigen der hierin genannten Komponenten der vorliegenden Erfindung in kabelgebundener oder kabelloser Signalverbindung stehen.

Fig. 3 zeigt außerdem eine medizinische Kraft U. Diese kann zum medizinischen Pflegepersonal gehören oder ein Arzt/eine Ärztin sein. In der Ausführungsform der Fig. 3 wird die medizinische Kraft U mittels der Sensoranordnung S erfasst. Dies kann beispielsweise mittels einer Kamera über Bewegungsmuster oder biometrische Daten erfolgen oder mittels Auslesens der Information zur Identität auf der Identifizierungsvorrichtung I, die die medizinische Kraft U optional mit sich führt. Alternativ oder ergänzend kann eine Stimmprobe mittels Mikrofons erfasst werden. Die von der Sensoranordnung S erfassten Informationen werden über die Lesevorrichtung A an die Berechtigungsvorrichtung B übermittelt, welche aufgrund der Berechtigungslage beispielsweise entscheiden kann, ob die Tür 700 für die medizinische Kraft U in Richtung des angedeuteten Pfeils geöffnet wird oder verschlossen bleibt oder, alternativ oder ergänzend, welche (keine, manche, alle) Funktionen der Behandlungsvorrichtung 1000 für die medizinische Kraft U zur Verfügung stehen.

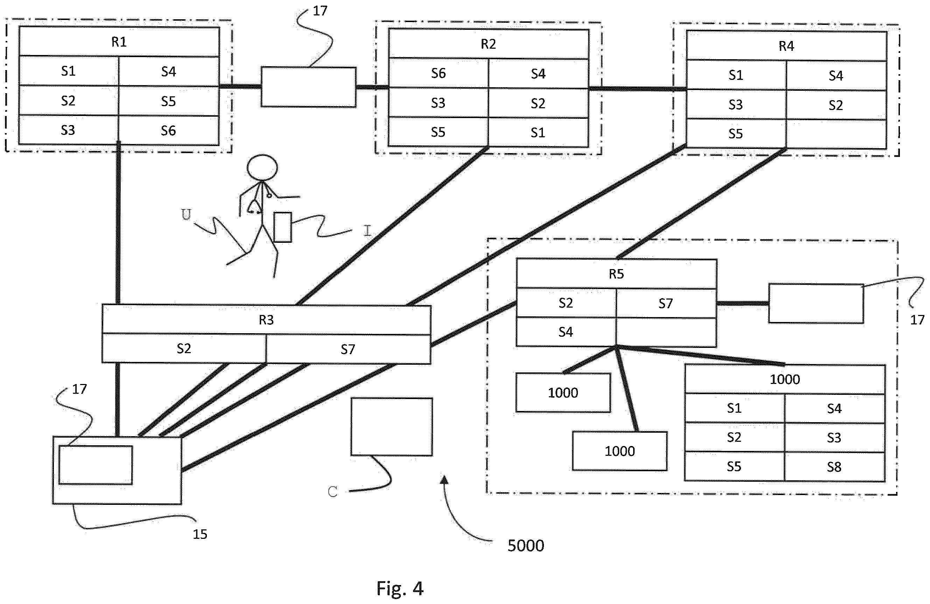

**Fig. 4** zeigt in vereinfachter Darstellung ein erfindungsgemäßes medizinisches Behandlungssystem 5000 in einer weiteren Ausführungsform.

Eine medizinische Kraft U kann durch verschiedene Verfahren identifiziert werden. Beispielsweise durch das Erfassen persönlicher Eigenschaften wie Bewegungsmuster, Stimme, Fingerabdruck, biometrischer Daten oder ihrer Irisstruktur. Außerdem durch das Abfragen persönlichen Wissens wie ein Passwort oder die Antwort auf eine Frage zum persönlichen Umfeld der medizinischen Kraft U. Ein weiteres Verfahren ist die Identifizierung der medizinischen Kraft U mittels einer Identifizierungsvorrichtung I, beispielsweise eine Magnet-oder Chipkarte, ein Badge oder ein Smartphone, die sie optional mit sich trägt.

Zur Erfassung dieser Informationen können verschiedene Sensoranordnungen S1 bis S8 einzeln oder in beliebiger Kombination verwendet werden. Zum Beispiel
- werden Fingerabdrücke mittels eines Fingerabdruckscanners S1,
- werden Bewegungsmuster, Gesten, biometrische Daten oder die Irisstruktur mittels einer Kamera S2 oder mittels einer Vorrichtung S8 zum Senden/Empfangen von Radarwellen,
- werden Magnet- oder Chipkarten mittels eines Kartenlesers S3,
- wird die Stimme mittels eines Mikrofons S4,
- wird persönliches Wissen mittels eines Touchscreens oder -pads oder einer Tastatur S5,
- Identifizierungsvorrichtungen I mittels Kamera S2, speziellen Erkennungsvorrichtungen S6, WiFi oder Bluetooth S7
erfasst und an die Lesevorrichtung A (siehe Fig. 1) übermittelt.

Fig. 4 zeigt in der linken unteren Ecke einen Server 15 des medizinisches Behandlungssystems 5000, auf dem in dieser Ausführungsform sowohl Lesevorrichtung A als auch die Berechtigungsvorrichtung B vorgesehen sein können. Ferner kann dieser Server 15 als Datenserver fungieren. Dies wird weiter unten ausgeführt.

Die Fig. 4 zeigt weiter exemplarisch zwei, vorzugsweise lokale, Datenbanken 17, auf denen ebenfalls Daten zur Feststellung einer Berechtigung hinterlegt sein können. Wird in diesen Bereichen, in der Fig. 4 beispielsweise Eingangsbereich R1, Umkleide R2 und Behandlungsraum R5, eine Person (lokal) identifiziert, so können mittels der auf den lokalen Datenbanken hinterlegten Daten schnell und einfach Berechtigungen ermittelt und entsprechende Aktoren vor Ort aktiviert werden.

Wenigstens eine lokale Datenbank 17 kann auch auf dem Server 15 vorliegen, welche beispielsweise alle Informationen enthält und nur einzelne für den Eingangsbereich R1, die Umkleide R2 und/oder den Behandlungsraum R5 relevante Informationen an die dortigen lokalen Datenbanken 17 übermittelt. Die lokalen Datenbanken 17 können aber auch im gesamten Netzwerk gespiegelt vorliegen, so dass alle Datenbanken identische Informationen aufweisen. Alternativ können die lokalen Datenbanken 17 auch unterschiedliche Informationen aufweisen, die für die Identifikation in den unterschiedlichen Bereichen notwendig sind.

Beispielsweise kann eine Berechtigungsvorrichtung B und/oder einer Lesevorrichtung A, welche für den Zugang zu beispielsweise einem Behandlungsraum R5 vorgesehen sind, mit einer lokalen Datenbank 17 verbunden sein, welche ausschließlich Informationen aufweist, welche konkrete medizinische Kraft U den beispielhaft genannten Behandlungsraum R5 betreten darf.

Eine medizinische Kraft U bewegt sich während ihrer Dienstzeit innerhalb der Klinik bzw. Praxis von Raum zu Raum. Hierfür durchquert sie sogenannte Übergangsbereiche R3 (Transition Areas), beispielsweise Gänge, Treppenhäuser, Vorräume, Zugänge und/oder dergleichen.

Der Eingangsbereich R1 umfasst einen Identifikationsbereich, in welchem Angehörige des medizinischen Personals wie die medizinische Kraft U identifiziert werden sollen. Die medizinische Kraft U kann beispielsweise mittels wenigstens einer der vorhandenen Sensoranordnungen, hier einem Fingerabdruckscanner S1, einer Kamera S2, einem Kartenleser S3, einem Mikrofon S4, einem Touchscreen oder - pad oder einer Tastatur S5 und/oder einer speziellen Erkennungsvorrichtung S6 für die Identifizierungsvorrichtungen I erfasst werden. Dabei kann die Erfassung mittels einer oder mehreren der Sensoranordnungen S1 bis S6 einzeln, nacheinander oder gleichzeitig, erfolgen. Die dabei erfasste Information wird mittels der Lesevorrichtung A in ein Netzwerk übermittelt, beispielsweise, um sie beispielsweise auf einem oder mehreren Server(n), in einer oder mehreren Datenbank(en) oder auf einem oder mehreren Datenspeicher(n) zu speichern und/oder, um sie mit Informationen, die beispielsweise auf dem Server 15 oder einer hiermit verbundenen Speichervorrichtung (in Fig. 4 nicht gezeigt) und/oder einer lokalen Datenbank 17 hinterlegt sein können, zu vergleichen. Unter den hinterlegten Informationen finden sich beispielsweise unter anderem Bilder, Stimmproben usw. der medizinischen Kraft U. Ferner kann hier eine Zuordnung der medizinischen Kraft U zu einer Personengruppe, z. B. einer Angestelltengruppierung, beispielsweise Ärzteschaft, Pflegepersonal eine andere Berufsgruppe, zu deren allgemeinen Gruppen-Berechtigungen und/oder zu individuellen Berechtigungen der konkreten Person erfolgen.

Der Datenserver ist vorzugsweise entweder ausschließlich von innerhalb der Klinik/Praxis zugänglich oder aber, ergänzend oder alternativ, von außerhalb.

Das Netzwerk, welches Teil des erfindungsgemäßen Behandlungssystem sein kann, umfasst beispielsweise, wie vorstehend bereits erwähnt, neben einem Datenserver eine Berechtigungsvorrichtung B, beispielsweise einen Rechner, welcher in Verbindung mit geeigneten Aktoren medizinischen Vorrichtungen wie Behandlungsvorrichtung oder Türen innerhalb der Klinik/Praxis partiell steuern oder anderweitig beeinflussen kann.

Die Informationen auf dem Datenserver, beispielsweise die Zugangsberechtigungen, die medizinischen Kräften U im Zusammenhang mit ihrer Identifizierung gewährt werden, sind vorzugsweise veränderlich, d. h. sie können z. B. erweitert oder widerrufen werden.

Ferner kann nach erfolgreicher Identifizierung der medizinischen Kraft U deren Identifizierungsvorrichtung I, sofern Erstere eine solche mit sich führt, derart modifiziert werden, dass die Berechtigungen darauf geändert sind, beispielsweise indem neue Informationen in oder auf diese geschrieben werden.

Alternativ oder ergänzend kann eine aktuelle Identifizierungsvorrichtung I an die medizinische Kraft U ausgegeben werden, die aktualisierte Berechtigungen enthält z. B. ein neuer Badge oder ein neuer Transponder oder dergleichen. Die Ausgabe kann ein Drucken, Ausdrucken, Abspeichern, Initialisieren, Programmieren usw. einer Identifizierungsvorrichtung, etwa einer Chipkarte, sein oder umfassen und z. B. an oder mittels eines hierzu geeigneten Ausgabe- und/oder Abgabeterminals C erfolgen.

Alternativ oder ergänzend kann das Ausgabe- und/oder Abgabeterminal C als Abgabeterminal vorgesehen sein. So kann es z. B. konfiguriert sein, um die Identifizierungsvorrichtung I aufzunehmen und auf ihr hinterlegte Inhalte mit den im Netzwerk hinterlegten Informationen abzugleichen, die auf der Identifizierungsvorrichtung I und/oder im Netzwerk, beispielsweise auf einem oder mehreren Server(n), in einer oder mehreren Datenbank(en) oder auf einem oder mehreren Datenspeicher(n), hinterlegten Inhalte zu modifizieren, zu aktualisieren und/oder Ähnliches.

Die Identifizierungsvorrichtung I kann eine der vorgenannten oder nachfolgenden Ausgestaltungen haben. Sie ist dazu vorgesehen, im Arbeitsablauf der medizinischen Kraft U, ein berührungsfreies und schnelles Identifizieren an relevanten Stellen, wie beispielsweise an Türen, Behandlungsvorrichtungen 1000, Diagnosevorrichtungen 2000 oder an weiteren Klinikvorrichtungen 3000 (siehe Fig. 1) und/oder an Zeiterfassungssystemen usw. zu ermöglichen.

Alternativ oder ergänzend kann das oben beschriebene Erfassen dann erfolgen, wenn die medizinische Kraft U einen Bereich betritt, der nur für Angehörige des Klinik-/Praxispersonals zugänglich ist, beispielsweise eine Umkleide R2. Hier wird der Zugang auf die oben beschriebene Art und Weise gewährt.

Insbesondere kann die Ausgabe einer Identifizierungsvorrichtung I an die Ausgabe von medizinischer Bekleidung gebunden sein. Hierzu kann die Identifizierungsvorrichtung I in die Bekleidung integriert sein. Zum Beispiel kann ein grafischer ID-Code an einem Arbeitskittel angebracht sein; ein weiteres Beispiel sind Arbeitsschuhe, die mit einem Radiosignaltransponder ausgestattet sind.

Ein Scannen der in die Bekleidung integrierten Identifizierungsvorrichtungen I in sensiblen Bereichen, beispielsweise mittels Kamera oder Radar, kann die medizinische Kraft U dort schnell und sicher identifizieren und mittels der Berechtigungseinrichtung B (siehe Fig. 1) Tätigkeiten erlauben, beispielsweise indem eine Tür entsperrt wird, oder verbieten, beispielsweise indem eine Tür verschlossen bleibt.

Diese Orte, die nur beschränkt zugänglich sind, beispielsweise die Umkleide R2, sind ebenfalls mittels des vorstehend beschriebenen medizinischen Behandlungssystems sensortechnisch abgedeckt, um Personen, die sich dort befinden als medizinische Kräfte identifizieren zu können. Dort befinden sich in manchen Ausführungsformen ebenfalls Ausgabe- und/oder Abgabeterminals für die Identifizierungsvorrichtungen I, wie oben ausgeführt.

Der Zugang zu einem Überwachungsraum R4 oder zu einem Behandlungsraum R5 wird nur medizinischen Kräften U gewährt, die sich an der Tür dieses Raumes identifiziert haben, beispielsweise indem die Sensoranordnungen S2, S4 und/oder S7 die medizinische Kraft U erfasst haben und die erfasste Information mittels der Berechtigungsvorrichtung B eine Zugangsberechtigung für diesen Raum ergeben hat. Ein analoges Vorgehen findet an den Behandlungsvorrichtungen 1000 statt.

Vorteilhafterweise werden in Überwachungsräumen R4 und Behandlungsräumen R5 berührungsfreie Identifizierungsverfahren verwendet werden, um unnötige Kontamination zu vermeiden.

Die vorliegende Erfindung ermöglicht die Ermittlung der Berechtigung der medizinischen Kraft U insbesondere bereits, wenn sich die medizinische Kraft U einer Tür nähert, dies kann unnötige Wartezeiten minimieren.

Für die Berechtigung, die die Berechtigungsvorrichtung B an den Behandlungsvorrichtungen 1000 vergibt, sollen hier drei Beispiele genannt werden:
(a) Es wird keine weitere Berechtigung der medizinischen Kraft U an den Behandlungsvorrichtungen 1000 geprüft, d. h. die Berechtigung den Raum zu betreten gilt als Berechtigung, die Behandlungsvorrichtungen 1000 auch zu bedienen.
(b) Berührungsfreie Berechtigungsermittlung mittels Scannens der Identifizierungsvorrichtung I der medizinischen Kraft U. Hierzu ist die Behandlungsvorrichtung 1000 mit einer Sensoranordnung (z. B. Kamera S2, Kartenleser S3, Radar S8) ausgestattet, um Informationen der Identifizierungsvorrichtung I empfangen zu können. Die Steuerung der Behandlungsvorrichtung 1000 lässt dann alle oder nur einen Teil der Funktionen zu. Die Funktionen der Behandlungsvorrichtung 1000, die der identifizierten medizinischen Kraft U zur Verfügung stehen sollen, werden mittels der lokalen Datenbank 17 bestimmt. Auf die lokale Datenbank 17 kann lokal von der Behandlungsvorrichtung 1000 oder über das Netzwerk mittels des entsprechenden Datenservers 15 zugegriffen werden. Auf diese Weise kann eine Benutzungshistorie in einer Datenbank hinterlegt werden, die festhalten kann, wer an der Behandlungsvorrichtung 1000 zu welchem Zeitpunkt welche Tätigkeit veranlasst hat.
(c) Identifizierung direkt an der Behandlungsvorrichtung 1000. Hierzu ist an der Behandlungsvorrichtung 1000 wenigstens eine Sensoranordnung (z. B. Fingerabdruckscanner S1, Kamera S2, Mikrofon S4, Touchscreen oder -pad oder Tastatur S5). Die Berechtigungsermittlung erfolgt dann analog zu Beispiel (b). In manchen Ausführungsformen ist die Behandlungsvorrichtung 1000 mit einem Radar ausgestattet, um (berührungsfreie) Gesten zu erkennen. Diese Gesten werden mit in der Datenbank hinterlegten Gesten verglichen, die wiederum eindeutig bestimmten Benutzern zugeordnet werden können. Die Berechtigung wird aufgrund der erfassten Geste und dem Ergebnis des Vergleichs ermittelt.

Innerhalb des Überwachungsraums R4 kann eine kabellose Verbindung zu bestimmten Behandlungsräumen R5 und den darin vorhandenen Behandlungsvorrichtungen 1000 aufgebaut werden. Medizinische Kräfte U, beispielsweise Ärzte und Ärztinnen, können dann kabellos Behandlungsparameter von bestimmten Behandlungsvorrichtungen 1000 auf einem Display im Überwachungsraum R4 überwachen. In manchen Ausführungsformen kann die medizinische Kraft U, nach erfolgter Identifizierung und bei entsprechender Berechtigung, in die Steuerung und/oder Regelung der Behandlungsvorrichtung 1000 eingreifen, d. h. die Behandlungsvorrichtung 1000 im Behandlungsraum R5 vom Überwachungsraum R4 aus steuern.

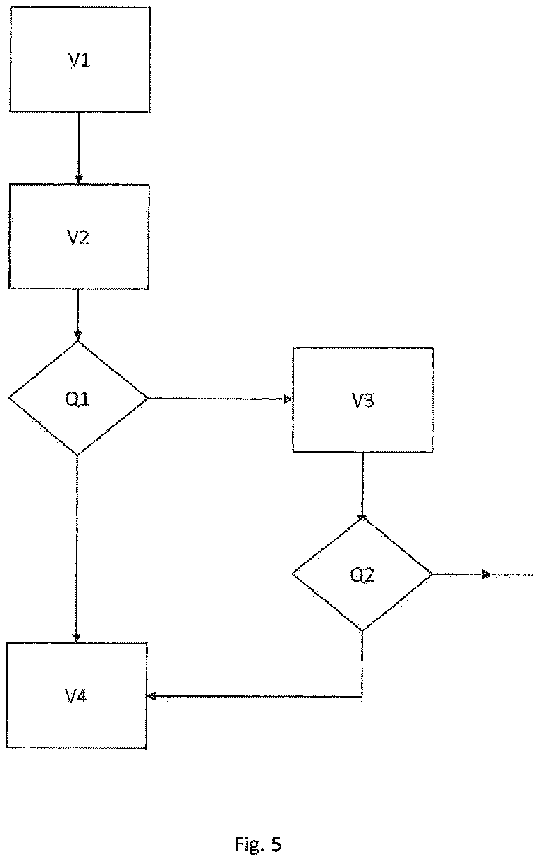

**Fig. 5** zeigt einen beispielshaften Ablaufplan einer Verwendung des erfindungsgemäßen medizinischen Behandlungssystems 5000 in einer beispielhaften Ausführungsform.

Die medizinische Kraft U trägt eine Identifizierungsvorrichtung I (siehe Fig. 1 bis Fig. 4), beispielsweise ein ID Badge, ein Armband, oder einen Transponder in oder an seinen Schuhen. Im Eingangsbereich einer Klinik/Praxis sind mehrere Sensoranordnungen angeordnet, beispielsweise ein Kartenleser S3, ein RFID-Lesegerät S8, eine Kamera S2 und/oder ein Fingerabdruckscanner S1 (nicht in Fig. 5 gezeigt, siehe Fig. 4).

Ein Schritt V1 repräsentiert das Erfassen oder Auslesen der Identifizierungsvorrichtung I am Eingang der Klinik/Praxis. Ist die Identifizierungsvorrichtung I z. B. eine Chipkarte, kann dies mittels eines Kartenlesers S3 erfolgen, ist sie z. B. ein Transponder, kann dies mittels eines RFID-Lesegeräts S8 erfolgen, usw.

In einem weiteren Schritt V2 soll das Gesicht der medizinischen Kraft U zu ihrer Identifizierung mittels Kamera erfasst werden.

Die Raute Q1 repräsentiert die Abfrage, ob das Gesicht erfolgreich erkannt und mittels der Lesevorrichtung A der zuvor identifizierten medizinischen Kraft U zugeordnet werden konnte.

Ist das Gesicht beispielsweise verdeckt, kann die Lesevorrichtung A aus den empfangenen Daten der Kamera S2 keine Identifizierung vornehmen. Dies ist mittels des Pfeils nach rechts dargestellt.

Aus diesem Grund wird in einem weiteren Schritt V3 die medizinische Kraft U aufgefordert, einen Fingerabdruckscan einzuleiten.

Die Raute Q2 repräsentiert die Abfrage, ob der Fingerabdruck erfolgreich gescannt und mittels der Lesevorrichtung A der zuvor identifizierten medizinischen Kraft U zugeordnet werden konnte.

Ist auch dies nicht der Fall, kann mittels weiterer Sensoranordnungen, die optional in diesem Bereich angeordnet sind, weiter versucht werden, die zuvor identifizierte medizinische Kraft U zu identifizieren. Alternativ kann erneut auf eine bereits durchgeführte Erkennungsart (hier also Gesichtserfassung, diesmal mit unverdecktem Gesicht oder ein Fingerabdruckscan, beispielsweise nach einer zwischengeschalteten Reinigung des Scanners) zurückgegriffen werden. Dies ist mit dem Pfeil nach rechts und den sich anschließenden unterbrochenen Strichen repräsentiert.

Hat sich die medizinische Kraft U auf eine der oben genannten Art und Weisen (also durch ein nicht verdecktes Gesicht oder einen erfolgreichen Fingerabdruckscan) eindeutig identifiziert (angedeutet jeweils durch den sich von den Rauten Q1 bzw. Q2 senkrecht nach unten ersteckenden Pfeil), kann sie, identifiziert durch ihre Identifizierungsvorrichtung I, die anstehende Arbeitsdauer oder Schicht mit den hierzu erforderlichen Berechtigungen antreten (Schritt V4 in Fig. 5): Mittels der Berechtigungsvorrichtung B werden dann z. B. ihre Arbeitsstunden automatisch mitgeschrieben, Türen können automatisch geöffnet werden, wenn die erforderlich Berechtigung der medizinischen Kraft U erkannt wird, Behandlungsvorrichtungen 1000 können von bedient werden, wenn die Identifizierungsvorrichtung I z. B. eine Armlänge oder weniger von ihnen entfernt ist, usw.

Wenn die maximale Arbeitszeit für diesen Tag erreicht ist, kann die medizinische Kraft U bis zum Beginn der nächsten Schicht z. B. am Zugang zum Behandlungsraum gehindert werden. Dies kann helfen, Fehler durch Übermüdung zu reduzieren oder zu eliminieren. Ferner kann anhand der so entzogenen Berechtigung später der Nachweis geführt werden, dass z. B. eine aufgrund unzulässig langer Arbeitstage möglicherweise unaufmerksame oder übermüdete medizinische Kraft U nicht an relevanten medizinischen Vorrichtungen tätig gewesen sein kann.

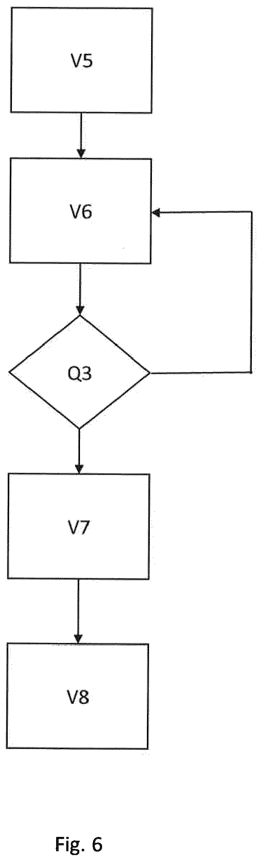

**Fig. 6** zeigt einen Ablaufplan eines Beispiels für die Verwendung des erfindungsgemäßen medizinischen Behandlungssystems 5000 in einer weiteren beispielhaften Ausführungsform.

Die medizinische Kraft U möchte Behandlungsparameter an einer Behandlungsvorrichtung 1000 auf einer Quarantänestation ändern.

In einem Schritt V5 führt die medizinische Kraft U deshalb eine vollständige Desinfektion durch und stattet sich mit der hierfür erforderlichen Sicherheitsbekleidung (z. B. Handschuhe, Maske, Kittel, Schutzbrille) aus und betritt den Behandlungsraum R5 (in Fig. 6 nicht gezeigt, siehe Fig. 4) auf der Quarantänestation. Die medizinische Kraft U kann sich an der Behandlungsvorrichtung 1000 aufgrund der von ihr getragenen Handschuhe nicht mittels Fingerabdrucks und wegen der aufgesetzten Gesichtsmaske nicht mittels Gesichtserkennung identifizieren. Eine Spracherkennung ist hier aufgrund des Dämpfungseffekts der Gesichtsmaske ebenfalls nicht möglich. Aus den vorgenannten Gründen ist hier eine Gestensteuerung mittels Radars das Mittel der Wahl.

Die Ausführung einer Geste durch die medizinische Kraft U, deren Erfassung mittels eines Radars durch die Lesevorrichtung A oder deren Weiterleitung an die Lesevorrichtung A ist durch einen Schritt V6 in Fig. 6 repräsentiert.

Mittels der an sie übermittelten Information kann die Lesevorrichtung A die medizinische Kraft U durch Vergleich der ausgeführten Geste mit bereits hinterlegten, vorbestimmten Gesten eindeutig identifizieren oder eben nicht. Diese Identifizierung ist in der Fig. 6 mittels einer Raute Q3 dargestellt.

Wurde die medizinische Kraft U eindeutig identifiziert (in Fig. 6 dargestellt durch einen Pfeil, ausgehend von der Raute Q3 senkrecht nach unten führend) kann die medizinische Kraft U mittels der Berechtigungsvorrichtung B entsprechend autorisiert und die Behandlungsvorrichtung 1000 zur Betätigung durch die medizinische Kraft U freigegeben werden.

Die medizinische Kraft U kann in einem Schritt V7 nun erforderliche Einstellungen oder Änderungen der Behandlungsparameter an der Behandlungsvorrichtung 1000 vornehmen, beispielsweise durch Spracherkennung. Die Behandlungsvorrichtung 1000 kann in einem solchen Fall dazu geeignet sein, Steuerbefehle für die Behandlung trotz Dämpfung der Maske eindeutig zu erkennen und ihre Umsetzung zu veranlassen. Eine Erkennung der Stimme ist an dieser Stelle nicht mehr notwendig, da sich die medizinische Kraft U bereits vorher wie oben beschrieben identifiziert hat.

Verlässt die medizinische Kraft U in einem Schritt V8 den Behandlungsraum R5 wieder, so wird die Behandlungsvorrichtung 1000 wieder gesperrt, um (dann unautorisierte) Eingaben bzw. Änderungen, beispielsweise durch den Patienten, nicht zuzulassen.

Wurde die medizinische Kraft nicht eindeutig identifiziert (siehe Raute Q3 in Fig. 6), so kann die medizinische Kraft U beispielsweise dazu veranlasst werden, die Geste erneut auszuführen. Dies ist in Fig. 6 repräsentiert durch einen waagrechten Pfeil von der Raute Q3 ausgehend nach rechts, der zum Schritt V6 zurückführt.

### Bezugszeichenliste

- 15: Server; Datenserver; Datenbankserver
- 17: lokale Datenbank

- 25: Zugabestelle für Heparin (optional)
- 29: venöse Blutkammer (optional)
- 31: Entlüftungseinrichtung

- 101: Blutpumpe
- 102: Dialysatablaufleitung, Effluentzulaufleitung
- 104: Dialysierflüssigkeitszulaufleitung
- 111: Pumpe für Substituat
- 121: Pumpe für Dialysierflüssigkeit
- 131: Pumpe für Dialysat oder Effluent in Effluentzulaufleitung
- 150: Steuer- oder Regelvorrichtung

- 200: Quelle mit Dialysierflüssigkeit
- 201: Quelle mit Substituat, optional

- 300: extrakorporaler Blutkreislauf
- 301: erste Leitung (arterieller Leitungsabschnitt)
- 301': (erster) Leitungsabschnitt am Patienten
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 305': (zweiter) Leitungsabschnitt am Patienten
- 306: (zweite) Schlauchklemme
- 400: Effluentbeutel
- 403: Effluentablaufleitung, Ausgussleitung

- 600: Ausguss

- 700: Tür

- 1000: Behandlungsvorrichtung
- 2000: Diagnosevorrichtung
- 3000: weitere Klinikvorrichtungen

- 5000: medizinisches Behandlungssystem

- A: Lesevorrichtung
- B: Berechtigungsvorrichtung
- C: Ausgabe- und/oder Abgabeterminal

- H1: Beutelheizung mit Beutel (Substituat)

- H2: Beutelheizung mit Beutel (Dialysierflüssigkeit)

- I: Identifizierungsvorrichtung

- P: Patient

- PS1, PS2: arterieller Drucksensor (optional)
- PS3: Drucksensor (optional)
- PS4: Drucksensor zum Messen des Filtratdrucks

- R1: Eingangsbereich
- R2: Umkleide
- R3: Übergangsbereich
- R4: Überwachungsraum
- R5: Behandlungsraum

- S: Sensoranordnung
- S': Sensoranordnung
- S1: Fingerabdruckscanner
- S2: Kamera
- S3: Kartenleser
- S4: Mikrofon
- S5: Touchscreen oder -pad; Tastatur
- S6: weitere Erkennungsvorrichtung
- S7: WiFi; Bluetooth
- S8: Radar; RFID-Lesegerät

- U: medizinische Kraft

- V1,..., V8: Ablaufschritte
- Q1,..., Q3: Abfragen bzgl. einer erfolgreichen Erfassung und Zuordnung/Identifizierung

## Patentansprüche

1. Medizinisches Behandlungssystem (5000), mit
- einer Lesevorrichtung (A) programmiert zum - vorzugsweise kontaktlosen - Erfassen von Informationen, welche einer Identifizierung der medizinischen Kraft (U) dienen, und programmiert zum Bereitstellen der erfassten Informationen;
- einer medizinischen Vorrichtung aus einer Gruppe von Elementen, welche aus einer oder mehreren Behandlungsvorrichtungen (1000) zum Behandeln eines Patienten, einer oder mehreren Diagnosevorrichtungen (2000) zum Untersuchen des Patienten und einer oder mehreren weitere Klinikvorrichtungen (3000), z. B. zum logistischen Ablauf des Klinik- oder Praxisbetriebs, besteht; und
- einer Berechtigungsvorrichtung (B) zum Erlauben oder Verbieten von einer oder mehreren Tätigkeiten mit oder an der medizinischen Vorrichtung und/oder einer oder mehreren Bewegungen der medizinischen Kraft (U) im Umfeld der medizinischen Vorrichtung in Abhängigkeit der mittels der Lesevorrichtung (A) bereitgestellten Informationen.

2. Medizinisches Behandlungssystem (5000) nach Anspruch 1, wobei die Lesevorrichtung (A) programmiert ist, um ein Bewegungsprofil, Gangschema oder Gesten der medizinischen Kraft (U) als Informationen zu erfassen.

3. Medizinisches Behandlungssystem (5000) nach einem der vorangegangenen Ansprüchen, weiter aufweisend wenigstens eine Identifizierungsvorrichtung (I) programmiert und/oder konfiguriert, um am Körper der medizinischen Kraft (U) getragen zu werden, wobei die Lesevorrichtung (A) programmiert ist, die Informationen durch Lesen der Identifizierungsvorrichtung (I) zu erfassen.

4. Medizinisches Behandlungssystem (5000) nach Anspruch 3, wobei die Lesevorrichtung (A) programmiert ist, um die erfassten Informationen, die insbesondere von der Identifizierungsvorrichtung (I) erfasst und übermittelt wurden, mit in der Lesevorrichtung (A) oder in einer Speichervorrichtung des medizinischen Behandlungssystems (5000) gespeicherten Informationen, insbesondere mit biometrischen Daten oder Signaturen, zu vergleichen.

5. Medizinisches Behandlungssystem (5000) nach einem der vorangegangenen Ansprüche, wobei die Lesevorrichtung (A) programmiert ist, um die Informationen durch Abfragen persönlichen Wissens der medizinischen Kraft (U) zu erfassen und mit in der Lesevorrichtung (A) oder in einer Speichervorrichtung des medizinischen Behandlungssystems (5000) gespeicherten Informationen zu vergleichen.

6. Medizinisches Behandlungssystem (5000) nach einem der vorangegangenen Ansprüche, wobei die Lesevorrichtung (A) programmiert ist, um basierend auf dem Ergebnis des Vergleichens die Identität der medizinischen Kraft (U) festzustellen.

7. Medizinisches Behandlungssystem (5000) nach einem der Ansprüche 3 bis 6, wobei
- die Identifizierungsvorrichtung (I) eine (Chip-oder Magnet-)Karte, eine Erkennungsmarke (Badge), ein Armband, ein Implantat, Bekleidung, eine Sehhilfe, Schuhe, grafische Drucke, aktive oder passive Transponder und/oder ein handgehaltenes Gerät, um die Informationen hieraus jeweils aus-oder einzulesen, ist oder umfasst.

8. Medizinisches Behandlungssystem (5000) nach einem der vorangegangenen Ansprüche, weiter aufweisend ein Ausgabe- und/oder Abgabeterminal (C) programmiert zum Erstellen, Beschreiben und/oder Auslesen einer Identifizierungsvorrichtung (I) zum Mitführen durch die medizinische Kraft (U), unter Aufbringen von Informationen auf die Identifizierungsvorrichtung (I) zum zeitlich beschränken und/oder räumlich beschränkten Erlauben oder Verbieten von einer oder mehreren Tätigkeiten mit oder an der medizinischen Vorrichtung und/oder einer oder mehreren Bewegungen der medizinischen Kraft (U) im Umfeld der medizinischen Vorrichtung.

9. Medizinisches Behandlungssystem (5000) nach einem der vorangegangenen Ansprüche, wobei
- die Behandlungsvorrichtung (1000) eine Infusionspumpe, ein Beatmungsgerät, eine extrakorporale Blutbehandlungsvorrichtung, insbesondere eine Dialysevorrichtung, Hämodialysevorrichtung, Hämofiltrationsvorrichtung oder Hämodiafiltrationsvorrichtung, insbesondere eine Vorrichtung für die akute, die chronische Nierenersatztherapie oder für die kontinuierliche Nierenersatztherapie (CRRT = continuous renal replacement therapy), ist oder umfasst.

10. Medizinisches Behandlungssystem (5000) nach einem der vorangegangenen Ansprüche, wobei
- die Diagnosevorrichtung (2000) ein Röntgengerät, ein Computertomograph, ein Magnetresonanztomograph (MRT), ein Ultraschallgerät, ein Gerät für die Endoskopie, ein Gerät zur Impedanzmessung, ein Gerät zur Thermografie, eine Gammakamera und/oder eine andere Vorrichtung für die Diagnose, insbesondere mittels bildgebender Verfahren, ist oder wenigstens eine(s) dieser Geräte oder Vorrichtungen umfasst.

11. Medizinisches Behandlungssystem (5000) nach einem der vorangegangenen Ansprüche, wobei
- die weitere medizinische Klinikvorrichtung (3000) ein Bildschirm zur medizinischen Überwachung, ein EKG-Gerät, ein medizinischer Kühlschrank oder ein medizinisches Kühlfach, eine Lagereinrichtung zum Lagern von medizinischen Einwegartikeln und/oder medizinischen Instrumenten ist oder wenigstens eine(s) dieser Geräte oder Vorrichtungen umfasst.

12. Medizinisches Behandlungssystem (5000) nach einem der vorangegangenen Ansprüche, wobei
- die Tätigkeiten Schritte zum Betätigen, Bedienen oder Verwenden der medizinischen Vorrichtung sind oder umfassen, insbesondere für einen bestimmungsgemäßen Gebrauch der medizinischen Vorrichtung.

13. Medizinisches Behandlungssystem (5000) nach einem der vorangegangenen Ansprüche, wobei
- die Bewegungen ein Sich-Annähern der medizinischen Kraft (U) an die medizinische Vorrichtung, ein Sich-Entfernen von der medizinischen Vorrichtung, ein Passieren, ein Betreten, ein Verlassen, ein sich Aufhalten im Umfeld, jeweils der medizinischen Kraft (U), sind oder umfassen.

14. Medizinisches Behandlungssystem (5000) nach einem der vorangegangenen Ansprüche, wobei
- das Umfeld eine vorbestimmte und/oder überwachte Fläche oder ein vorbestimmter und/oder überwachter Raum der Klinik, insbesondere ein Eingangsbereich, ein allgemein zugänglicher Bereich, ein Bereich, der nur mit Berechtigung betreten werden dürfen, ein Bereich, in welchem sich durch die Anwesenheit Arbeitsbeginn und/oder -ende definieren lassen, ein oder mehrere Behandlungsräume, einen oder mehrere Überwachungsräume, ein Raum, in welchem die medizinische Vorrichtung der und/oder der Wegebereich von einem zum anderen der vorgenannten Räume ist, oder jeweils umfasst.

15. Medizinisches Behandlungssystem (5000) nach einem der vorangegangenen Ansprüche, weiter aufweisend
- eine Schreibvorrichtung (W), programmiert zum - vorzugsweise kontaktlosen - Schreiben oder Überschreiben von Informationen auf die/der Identifizierungsvorrichtung (I) unter Erstellen von für die Lesevorrichtung (A) lesbaren Informationen.

16. Medizinisches Behandlungssystem (5000) nach einem der vorangegangenen Ansprüche, weiter aufweisend
- eine Schreibvorrichtung (W), programmiert zum - vorzugsweise kontaktlosen - Schreiben oder Überschreiben von Informationen auf die/der Berechtigungsvorrichtung (B), die dem Erlauben oder Verbieten von einer oder mehreren Tätigkeiten mit oder an der medizinischen Vorrichtung und/oder einer oder mehreren Bewegungen der medizinischen Kraft (U) im Umfeld der medizinischen Vorrichtung dienen.
